# EUROPEAN PATENT SPECIFICATION

(11) **EP 0 541 168 B1**
(45) Date of publication and mention of the grant of the patent: **11.03.1998**
(21) Application number: 92203357.6
(22) Date of filing: 02.11.1992
(51) Int. Cl.: C07D 217/26, C07D 241/04, C07D 409/12, A61K 31/47, A61K 31/495

(54) **HIV protease inhibitors useful for the treatment of aids**
HIV-Protease-Inhibitoren verwendbar in der AIDS-Behandlung
Inhibiteurs d'HIV-protéase utilisables dans le traitement du SIDA

(30) Priority: 08.11.1991 US 789508; 15.05.1992 US 883825
(43) Date of publication of application: 12.05.1993
(73) Proprietor: Merck & Co., Inc., Rahway New Jersey 07065-0900 (US)
(72) Inventor: Vacca, Joseph P., Telford, PA 18969 (US); Holloway, M. Katharine, Lansdale, PA 19446 (US); Dorsey, Bruce D., Harleysville, PA 19438 (US); Hungate, Randall W., Lansdale, PA 19446 (US); Guare, James P., Quackerstown, PA 18951 (US)
(74) Representative: Barrett-Major, Julie Diane

(56) References cited:
- EP-A- 0 432 695
- EP-A- 0 434 365
- JOURNAL OF MEDICINAL CHEMISTRY vol. 34, no. 3, March 1991, WASHINGTON US pages 1228 - 1230 TERRY A. LYLE 'Benzocycloalkyl amines as novel c-termini for HIV protease inhibitors'
- SCIENCE vol. 248, 20 April 1990, LANCASTER, PA US pages 358 - 361 NOEL A. ROBERTS ET AL 'Rational design of peptide-based HIV proteinase inhibitors'

## Description

The present invention is concerned with compounds which inhibit the protease encoded by human immunodeficiency virus (HIV) or pharmaceutically acceptable salts thereof and are of value in the prevention of infection by HIV, the treatment of infection by HIV and the treatment of the resulting acquired immune deficiency syndrome (AIDS). It also relates to pharmaceutical compositions containing the compounds and to a method of use of the present compounds and other agents for the treatment of AIDS and viral infection by HIV.

### BACKGROUND OF THE INVENTION

A retrovirus designated human immunodeficiency virus (HIV) is the etiological agent of the complex disease that includes progressive destruction of the immune system (acquired immune deficiency syndrome; AIDS) and degeneration of the central and peripheral nervous system. This virus was previously known as LAV, HTLV-III, or ARV. A common feature of retrovirus replication is the extensive post-translational processing of precursor polyproteins by a virally encoded protease to generate mature viral proteins required for virus assembly and function. Inhibition of this processing prevents the production of normally infectious virus. For example, Kohl, N.E. et al., Proc. Nat'l Acad. Sci. 85, 4686 (1988) demonstrated that genetic inactivation of the HIV encoded protease resulted in the production of immature, non-infectious virus particles. These results indicate that inhibition of the HIV protease represents a viable method for the treatment of AIDS and the prevention or treatment of infection by HIV.

The nucleotide sequence of HIV shows the presence of a pol gene in one open reading frame [Ratner, L. et al., Nature, 313, 277(1985)]. Amino acid sequence homology provides evidence that the pol sequence encodes reverse transcriptase, an endonuclease and an HIV protease [Toh, H. et al., EMBO J. 4, 1267 (1985); Power, M-D- et al., Science, 231, 1567 (1986); Pearl, L.H. et al., Nature 329, 351 (1987)]. Applicants demonstrate that the compounds of this invention are inhibitors of HIV protease.

EP-A-434365 discloses compounds of formula :

A-G-B-B-J

where A is an amine protecting group or methane, G is a dipeptide isostefe substituted with a basic nitrogen, B is an amino acid or analogue thereof and J is a small terminal group for the inhibition of HIV protease.

### BRIEF DESCRIPTION OF THE INVENTION

Compounds of formula I, as herein defined, are disclosed. These compounds are useful in the inhibition of HIV protease, the prevention of infection by HIV, the treatment of infection by HIV and in the treatment of AIDS, either as compounds, pharmaceutically acceptable salts, pharmaceutical composition ingredients, whether or not in combination with other antivirals, immunomodulators, antibiotics or vaccines. Methods of treating AIDS, methods of preventing infection by HIV, and methods of treating infection by HIV are also disclosed.

Some abbreviations that may appear in this application are as follows.

| ABBREVIATTONS | |
|---|---|
| Designation | Protecting Group |
| BOC (Boc) | t-butyloxycarbonyl |
| CBZ (Cbz) | benzyloxycarbonyl(carbobenzoxy) |
| TBS (TBDMS) | t-butyl-dimethylsilyl |
| | |

| | Activating Group |
|---|---|
| HBT(HOBT or HOBt) | 1-hydroxybenzotriazole |
| | hydrate |
| | |

| Designation | Coupling Reagent |
|---|---|
| BOP reagent | benzotriazol-1-yloxytris-(dimethylamino)phosphonium hexafluorophosphate |
| BOP-C1 | bis(2-oxo-3-oxazolidinyl) phosphinic chloride |
| EDC | 1-ethyl-3-(3-dimethylaminopropyl) carbodiimide hydrochloride |
| | |

| | Other |
|---|---|
| (BOC)₂O (BOC₂O) | di-t-butyl dicarbonate |
| n-BU₄N⁺F⁻ | tetrabutyl ammonium |
| | fluoride |
| nBuLi (n-Buli) | n-butyllithium |
| DMF | dimethylformamide |
| Et₃N | triethylamine |
| EtOAc | ethyl acetate |
| TFA | trifluoroacetic acid |
| DMAP | dimethylaminopyridine |
| DME | dimethoxyethane |
| LDA | lithium diisopropylamide |
| THF | tetrahydrofuran |
| | |

| | Amino Acid |
|---|---|
| Ile | L-isoleucine |
| Val | L-valine |

### DETAILED DESCRIPTION OF THE INVENTION AND PREFERRED EMBODIMENTS

This invention is concerned with compounds of formula I, combinations thereof, or pharmaceutically acceptable salts thereof, in the inhibition of HIV protease, the prevention or treatment of infection by HIV and in the treatment of the resulting acquired immune deficiency syndrome (AIDS). Compounds of formula I are defined as follows: 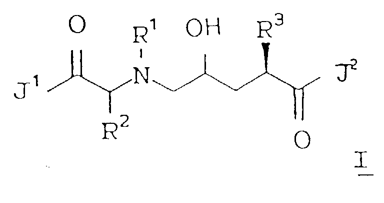 wherein
R is hydrogen or C₁₋₄ alkyl;
R¹ and R² can be joined together to form with the nitrogen to which R¹ is attached a 3 to 10 membered monocyclic or bicyclic saturated ring system which consists of the nitrogen to which R¹ is attached and from 2 to 9 carbon atoms, and is unsubstituted or substituted with
   1) -W-aryl, or
   2) 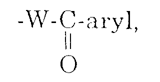 wherein W is -O-, -S- or -NH-; or
R¹ and R² can be joined together to form with the nitrogen to which R¹ is attached a 3 to 10 membered monocyclic or bicyclic saturated ring system which consists of the nitrogen to which R¹ is attached, from 1 to 8 carbon atoms and one of 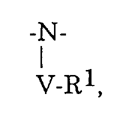 wherein V is absent or 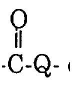 or -SO₂-Q-, R¹ is
   1) hydrogen,
   2) -C₁₋₄ alkyl unsubstituted or substituted with one or more of
      a) halo,
      b) hydroxy,
      c) C₁₋₃ alkoxy,
      d) aryl unsubstituted or substituted with one or more of C₁₋₄alkyl, hydroxy or aryl,
      e) -W-aryl or -W-benzyl, wherein W is -O-, -S-, or -NH-,
      f) a 5-7 membered cycloalkyl group unsubstituted or substituted with one or more of
         i) halo,
         ii) hydroxy,
         iii) C₁₋₃ alkoxy, or
         iv) aryl,
      g) heterocycle unsubstituted or substituted with one or more of hydroxy, C₁₋₄alkyl optionally substituted with hydroxy, or Boc,
      h) 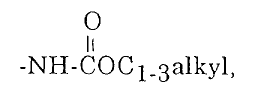
      i) 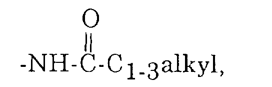
      j) -NH-SO₂C₁₋₃alkyl,
      k) -NR₂,
      l) -COOR, or
      m) -((CH₂)ₘO)ₙR wherein m is 2-5 and n is zero, 1, 2 or 3, or
   3) aryl, unsubstituted or substituted with one or more of
      a) halo,
      b) hydroxy,
      c) -NO₂ or -NR₂,
      d) C₁₋₄alkyl,
      e) C₁₋₃ alkoxy, unsubstituted or substituted with one or more of -OH or C₁₋₃ alkoxy
      f) -COOR,
      g) 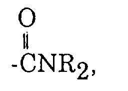
      h) -CH₂NR₂,
      i) 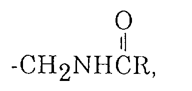
      j) -CN,
      k) -CF₃,
      l) 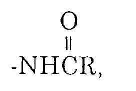
      m) aryl C₁₋₃ alkoxy,
      n) aryl,
      o) -NRSO₂R,
      p) -OP(O)(ORₓ)₂, or
      q) -R⁵, as defined below; and wherein Q is absent or -O-, -NR-, or heterocycle optionally substituted with -C₁₋₄alkyl,
R³ is benzyl, unsubstituted or substituted with one or more of (1) hydroxy, (2) C₁₋₃ alkoxy substituted with one or more of -OH or (3) 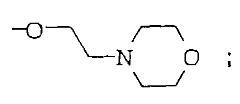
Rₓ is H or aryl;
R⁵ is
   1) -W-(CH₂)ₘ-NR⁶R⁷
      wherein W is as defined above,
      m is 2-5, and
      R⁶ and R⁷ are independently
         a) hydrogen,
         b) C₁₋₆ alkyl, unstibstituted or substituted with one or more of
            i) C₁₋₃ alkoxy,
            ii) -OH, or
            iii) -NR₂,
         c) the same or different and joined together to form a 5-7 member heterocycle, such as morpholino containing up to two additional heteroatoms selected from 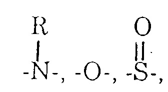 -S-, or -SO₂-, the heterocycle optionally substituted with C₁₋₄ alkyl, or
         d) aromatic heterocycle unsubstituted or substituted with one or more of
            i) C₁₋₄ alkyl, or
            ii) -NR₂
   2) -(CH₂)_{q}-NR⁶R⁷ wherein q is 1-5, and R⁶ and R⁷ are defined above, except that R⁶ or R⁷ are not H or unsubstituted C₁₋₆ alkyl, or
   3) benzofuryl indolyl, azacycloalkyl, azabicyclo C₇₋₁₁ cycloalkyl, or benzopiperidinyl, unsubstituted or substituted with C₁₋₄ alkyl;
J¹ is -NH-C₁₋₄alkyl; and
J² is

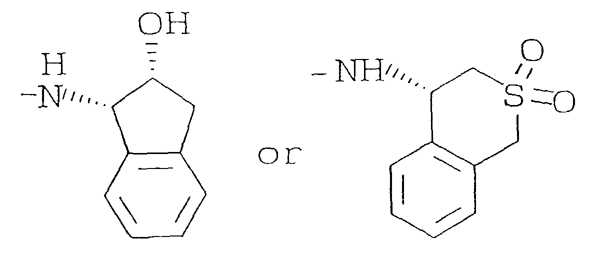

The present invention also provides a compound of formula 27: 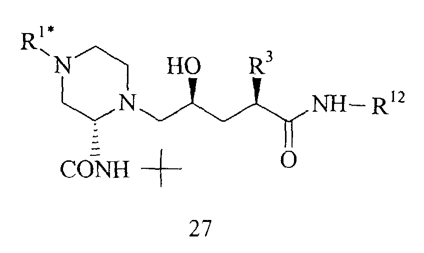 wherein R¹*, R³ and R¹² are as hereinbefore defined except that when V is absent or represents -SO₂-Q- then R¹ is not hydrogen or a group, other than an aryl group, with a free hydroxy substituent, or a pharmaceutically acceptable salt thereof.

The present invention further provides a process for the preparation of a compound of formula 27: 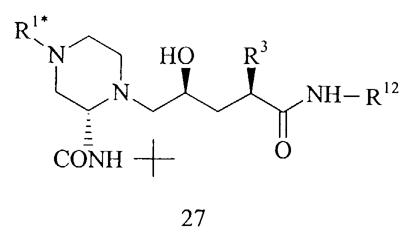 wherein R¹*, R³ and R¹² are as hereinbefore defined except that when V is absent or represents -SO₂-Q- then R¹ is not hydrogen or a group, other than an aryl group, with a free hydroxy substituent, which comprises reacting a compound of formula 28: 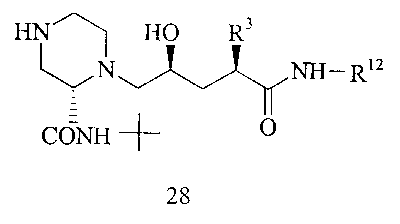 wherein R³ and R¹² are as defined above with a compound of formula R¹*-X or R¹*SO₂Cl, where R¹* is as defined above and X is Cl, Br or I, or using standard amide coupling techniques.

In one embodiment the compound of formula 28 is reacted with a compound of formula R¹*-X.

Preferably R³ and R¹² in the compounds of formulae 27 and 28 are (R)-benzyl and 2(R)-hydroxy-1(S)-indanyl respectively. Preferably the compound of formula 28 is N-2(2(R)-hydroxy- 1(S)-indanyl)-2(R)-phenylmethyl-4(S)-hydroxy-5-(1-(2(S)-N-(t-butylcarboxamido)piperazinyl))-pentaneamide.

The most preferred compounds of this invention are compounds A through H and J, shown below. 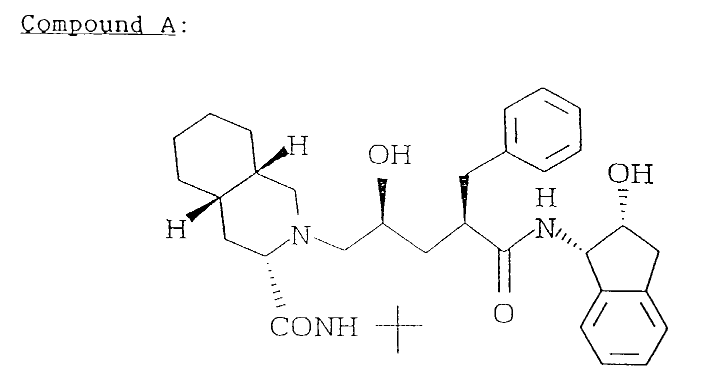 N-(2(R)-hydroxy-1(S)-indanyl)-2(R)-phenylmethyl-4(S)-hydroxy-5-(2-(3(S)-N'-(t-butylcarboxamido)-(4aS,8aS)-decahydroisoquinoline)yl)-pentaneamide, 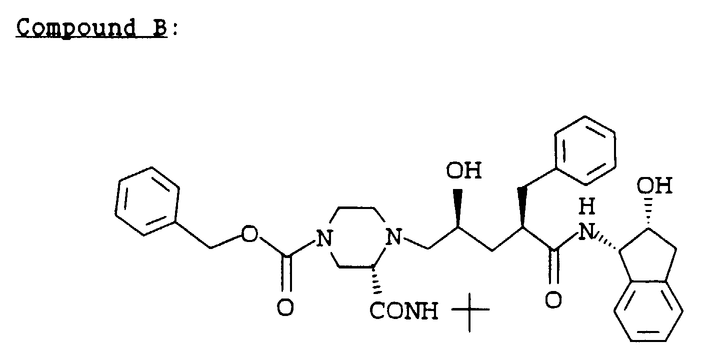 N-(2(R)-hydroxy-1(S)-indanyl)-2(R)-phenylmethyl-4(S)-hydroxy-5-(1-(4-carbobenzyloxy-2(S)-N'-(t-butylcarboxamido)-piperazinyl))-pentaneamide, 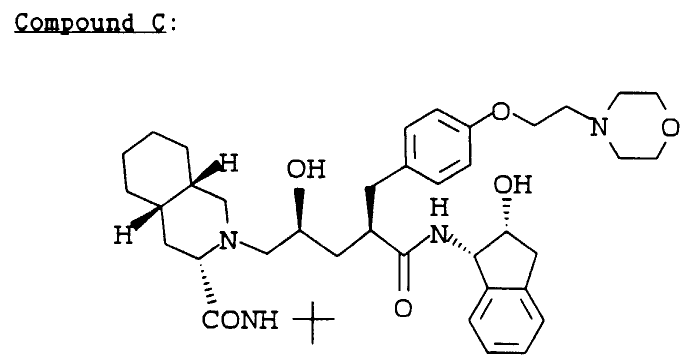 N-(2(R)-hydroxy-1(S)-indanyl)-2(R)-((4-(2-(4-morpholinyl)ethoxy)phenyl)methyl)-4(S)-hydroxy-5-(2-(3(S)-N'-(t-butylcarboxamido)-(4aS,8aS)-decahydroisoquinoline)yl)-pentaneamide, 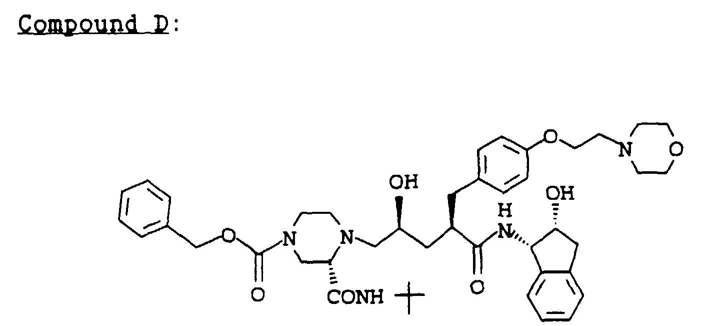 N-(2(R)-hydroxy-1(S)-indanyl)-2(R)-((4-(2-(4-morpholinyl)ethoxy)phenyl)methyl)-4(S)-hydroxy-5-(1-(4-carbobenzyloxy-2(S)-N'-(t-butylcarboxamido)piperazinyl))-pentaneamide, 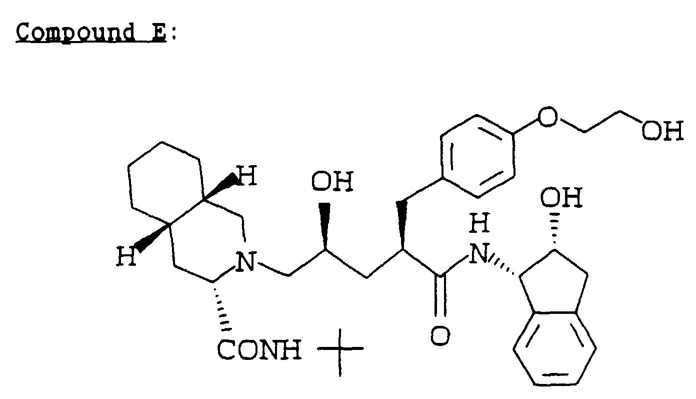 N-(2(R)-hydroxy-1(S)-indanyl)-2(R)-((4-((2-hydroxy)-ethoxy)phenyl)methyl)-4(S)-hydroxy-5-(2-(3(S)-N'-(t-butylcarboxamido)-(4aS,8aS)-decahydroisoquinoline)yl)-pentaneamide, 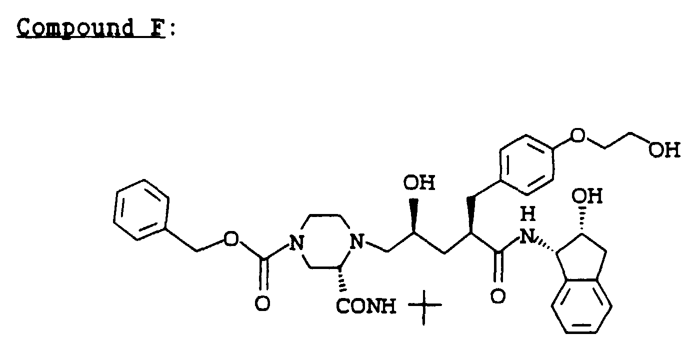 N-(2(R)-hydroxy-1(S)-indanyl)-2(R)-((4-((2-hydroxy)ethoxy)phenyl)methyl)-4(S)-hydroxy-5-(1-(4-carbobenzyloxy-2(S)-N'-(t-butylcarboxamido)piperazinyl))-pentaneamide, 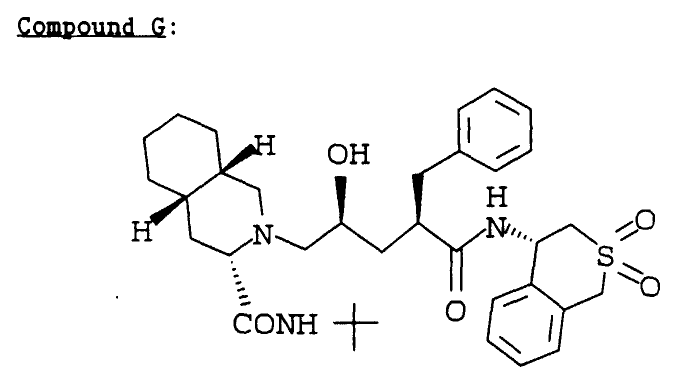 N-(4(S)-3,4-dihydro-1H-2,2-dioxobenzothiopyranyl)-2(R)-phenylmethyl-4(S)-hydroxy-5-(2-(3(S)-N'-(t-butylcarboxamido)-(4aS,8aS)-decahydroisoquinoline)yl)pentaneamide, 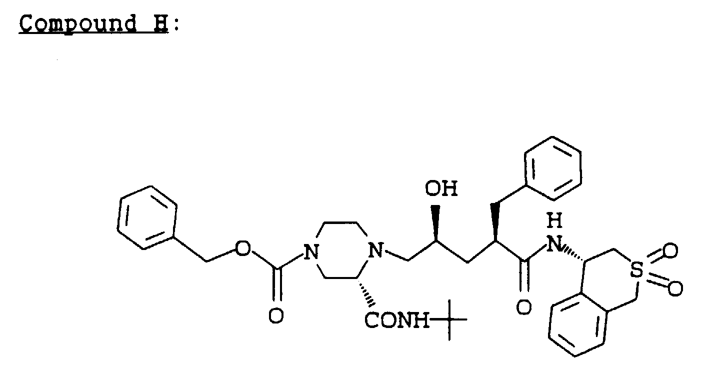 N-(4(S)-3,4-dihydro-1H-2,2-dioxobenzothiopyranyl)-2(R)-phenylmethyl-4(S)-hydroxy-5-(1-(4-carbobenzyloxy-2(S)-N'-(t-butylcarboxamido)-piperazinyl))pentaneamide, 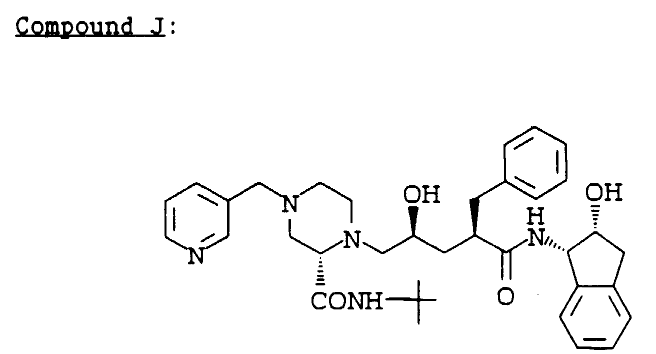 N-(2(R)-hydroxy-1(S)-indanyl)-2(R)-phenylmethyl4(S)-hydroxy-5-(1-(4-(3-pyridylmethyl)-2(S)-N'-(t-butylcarboxamido)-piperazinyl))-pentaneamide.

Novel compounds of the present invention also include but are not limited to the following compounds:
N-(2(R)-hydroxy-1(S)-indanyl)-2(R)-phenylmethyl-4-(S)-hydroxy-5-(1-(4-(3-phenylpropionyl)-2(S)-N'-(t-butylcarboxamido)-piperazinyl))-pentaneamide,
N-(2(R)-hydroxy-1(S)-indanyl)-2(R)-phenylmethyl-4-(S)-hydroxy-5-(1-(4-benzoyl-2(S)-N'-(t-butylcarboxamido)-piperazinyl))-pentaneamide,
N-(2(R)-hydroxy-1(S)-indanyl)-2(R)-phenylmethyl-4-(S)-hydroxy-5-(1-(4-(3-phenylpropyl)-2(S)-N'-(t-butylcarboxamido)-piperazinyl))-pentaneamide,
N-(2(R)-hydroxy-1(S)-indanyl)-2(R)-((4-(2-(4-morpholinyl)ethoxy)phenyl)methyl)-4(S)-hydroxy-5-(1-(4-(3-phenylpropionyl)-2(S)-N'-(t-butylcarboxamido)piperazinyl))-pentaneamide,
N-(2(R)-hydroxy-1(S)-indanyl)-2(R)-((4-(2-(4-morpholinyl)ethoxy)phenyl)methyl)-4(S)-hydroxy-5-(1-(4-benzoyl-2(S)-N'-(t-butylcarboxamido)-piperazinyl))pentaneamide,
N-(2(R)-hydroxy-1(S)-indanyl)-2(R)-((4-(2-(4-morpholinyl)ethoxy)phenyl)methyl)-4(S)-hydroxy-5-(1-(4-(3-phenylpropyl)-2(S)-N'-(t-butylcarboxamido))-piperazinyl)-pentaneamide,
N-(2(R)-hydroxy-1(S)-indanyl)-2(R)-((4-((2-hydroxy)ethoxy)phenyl)methyl)-4(S)-hydroxy-5-(1-(4-(3-phenylpropionyl)-2(S)-N'-(t-butylcarboxamido)-piperazinyl))pentaneamide,
N-(2(R)-hydroxy-1(S)-indanyl)-2(R)-((4-((2-hydroxy)ethoxy)phenyl)methyl)-4(S)-hydroxy-5-(1-(4-benzoyl-2(S)-N'-(t-butylcarboxamido)-piperazinyl))pentaneamide,
N-(2(R)-hydroxy-1(S)-indanyl)-2(R)-((4-((2-hydroxy)ethoxy)phenyl)methyl)-4(S)-hydroxy-5-(1-(4-(3-phenylpropyl)-2(S)-N'-(t-butylcarboxamido))-piperazinyl)pentaneamide,
N-(4(S)-3,4-dihydro-1H-2,2-dioxobenzothiopyranyl)-2-(R)-phenylmethyl-4(S)-hydroxy-5-(1-(4-(3-phenylpropionyl)-2(S)-N'-(t-butylcarboxamido)-piperazinyl))pentaneamide,
N-(4(S)-3,4-dihydro-1H-2,2-dioxobenzothiopyranyl)-2-(R)-phenylmethyl-4(S)-hydroxy-5-(1-(4-benzoyl-2(S)-N'-(t-butylcarboxamido)-piperazinyl))-pentaneamide,
N-(4(S)-3,4-dihydro-1H-2,2-dioxobenzothiopyranyl)-2-(R)-phenylmethyl-4(S)-hydroxy-5-(1-(4-(3-phenylpropyl)-2(S)-N'-(t-butylcarboxamido))-piperazinyl)pentaneamide, or
(4(S)-3,4-dihydro-1H-2,2-dioxobenzothiopyranyl)-2(R)-phenylmethyl-4(S)-amino-5-(1-(4-carbobenzyloxy-2(S)-N'-(t-butylcarboxamido)-piperazinyl))-pentaneamide.

The compounds of the present invention, may have asymmetric centers and occur as racemates, racemic mixtures and as individual diastereomers, or enantiomers with all isomeric forms being included in the present invention.

When any variable (e.g., aryl, heterocycle, R, R¹, R², n, etc.) occurs more than one time in any constituent or in formula I, its definition on each occurrence is independent of its definition at every other occurrence. Also, combinations of substituents and/or variables are permissible only if such combinations result in stable compounds.

As used herein except where noted, "alkyl" is intended to include both branched- and straight-chain saturated aliphatic hydrocarbon groups having the specified number of carbon atoms (Me is methyl, Et is ethyl, Pr is propyl, Bu is butyl); "alkoxy" represents an alkyl group of indicated number of carbon atoms attached through an oxygen bridge; and "cycloalkyl" is intended to include saturated ring groups, such as cyclopropyl, cyclobutyl, cyclopentyl, cyclohexyl (Cyh) and cycloheptyl. "Alkenyl" is intended to include hydrocarbon groups of either a straight or branched configuration with one or more carbon-carbon double bonds which may occur in any stable point along the chain, such as ethenyl, propenyl, butenyl, pentenyl, and the like. "Alkynyl" is intended to include hydrocarbon groups of either a straight or branched configuration with one or more carbon-carbon triple bonds which may occur in any stable point along the chain, such as ethynyl, propynyl, butynyl, pentynyl, and the like. "Halo", as used herein, means fluoro, chloro, bromo and iodo; and "counterion" is used to represent a small, single negatively-charged species, such as chloride, bromide, hydroxide, acetate, trifluroacetate, perchlorate, nitrate, benzoate, maleate, tartrate, hemitartrate, benzene sulfonate, and the like.

As used herein, with exceptions as noted, "aryl" is intended to mean phenyl (Ph) or naphthyl. "Carbocyclic" is intended to mean any stable 5- to 7-membered carbon ring or 7- to 10-membered bicyclic carbon ring any ring of which may be saturated or unsaturated.

The term heterocycle or heterocyclic, as used herein except where noted, represents a stable 5- to 7-membered mono- or bicyclic or stable 7- to 10-membered bicyclic heterocyclic ring system any ring of which may be saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. The heterocyclic ring may be attached at any heteroatom or carbon atom which results in the creation of a stable structure. Examples of such heterocyclic elements include piperidinyl, piperazinyl, 2-oxopiperazinyl, 2-oxopiperidinyl, 2-oxopyrrolodinyl, 2-oxoazepinyl, azepinyl, pyrrolyl, 4-piperidonyl, pyrrolidinyl, pyrazolyl, pyrazolidinyl, imidazolyl, imidazolinyl, imidazolidinyl, pyridyl, pyrazinyl, pyrimidinyl, pyridazinyl, oxazolyl, oxazolidinyl, isoxazolyl, isoxazolidinyl, morpholinyl, thiazolyl, thiazolidinyl, isothiazolyl, quinuclidinyl, isothiazolidinyl, indolyl, quinolinyl, isoquinolinyl, benzimidazolyl, thiadiazoyl, benzopyranyl, benzothiazolyl, benzoxazolyl, furyl, tetrahydrofuryl, tetrahydropyranyl, thienyl, benzothienyl, thiamorpholinyl, thiamorpholinyl sulfoxide, thiamorpholinyl sulfone, and oxadiazolyl. Morpholino is the same as morpholinyl.

The pharmaceutically-acceptable salts of the compounds of Formula I (in the form of water- or oil-soluble or dispersible products) include the conventional non-toxic salts or the quaternary ammonium salts which are formed, e.g., from inorganic or organic acids or bases. Examples of such acid addition salts include acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, pamoate, pectinate, persulfate, 3-phenylpropionate, picrate, pivalate, propionate, succinate, tartrate, thiocyanate, tosylate, and undecanoate. Base salts include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine, lysine, and so forth. Also, the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others.

Schemes I-III for preparing the novel compounds of this invention are presented below. Tables I and II which follow the schemes illustrate the compounds that can be synthesized by Schemes I-III, but Schemes I-III are not limited by the compounds in the tables nor by any particular substituents employed in the schemes for illustrative purposes. The examples specifically illustrate the application of the following schemes to specific compounds.

Amide couplings used to form the compounds of this invention are typically performed by the carbodiimide method with reagents such as hexylcarbodiimide, or 1-ethyl-3-(3-dimethylaminopropyl) carbodiimide. Other methods of forming the amide or peptide bond include, but are not limited to the synthetic routes via an acid chloride, azide, mixed anhydride or activated ester. Typically, solution phase amide coupling are performed, but solid-phase synthesis by classical Merrifield techniques may be employed instead. The addition and removal of one or more protecting groups is also typical practice.

Additional related information on synthetic background is contained in EPO 0337714.

One method for producing formula I compounds is provided by Scheme I. Dihydro-5(S)-(tert-butyldimethylsilyloxymethyl)-3(2H)-furanone (compound 1 below) is prepared by standard methods known in the art from commercially available dihydro-5(S)-(hydroxymethyl)-2(3H)-furanone. After alkylation of compound 1 to form compound 2, the protecting group of lactone 2 is removed with aqueous HF to afford compound 3.

The alcohol group of 3 is activated by conversion into a leaving group such as mesylate, tosylate or trifylate by treating the alcohol with a sulfonyl chloride or sulfonic anhydride, such as trifluoromethanesulfonic anhydride, in the presence of a hindered amine base such as triethylamine, diethyl isopropylamine or 2,6 lutidine, to afford a compound such as compound 4. The leaving group of compound 4 is displaced by an amine 5, such as N'-t-butyl-(4aS,8aS)-(decahydroisoquinoline)-3(S)-carboxamide, in a high boiling solvent such as DMF or xylene to produce a compound such as 6. A trifluoromethanesulfonyloxy group can be displaced by an amine at room temperature in a solvent such as isopropanol by treatment with N,N-diisopropylethylamine.

Compound 6 is hydrolyzed with aqueous lithium or sodium hydroxide and the resultant hydroxy acid 7 is converted into a protected hydroxy acid 8. The hydroxyl group is conveniently protected with a standard silyl protecting group such as t-butyldimethyl silyl or t-butyldiphenyl silyl.

The protected hydroxy-acid 8 is then coupled to the desired R¹² amine to produce compound 9, and the silyl protecting group is removed with fluoride ion to arrive at compound 10.

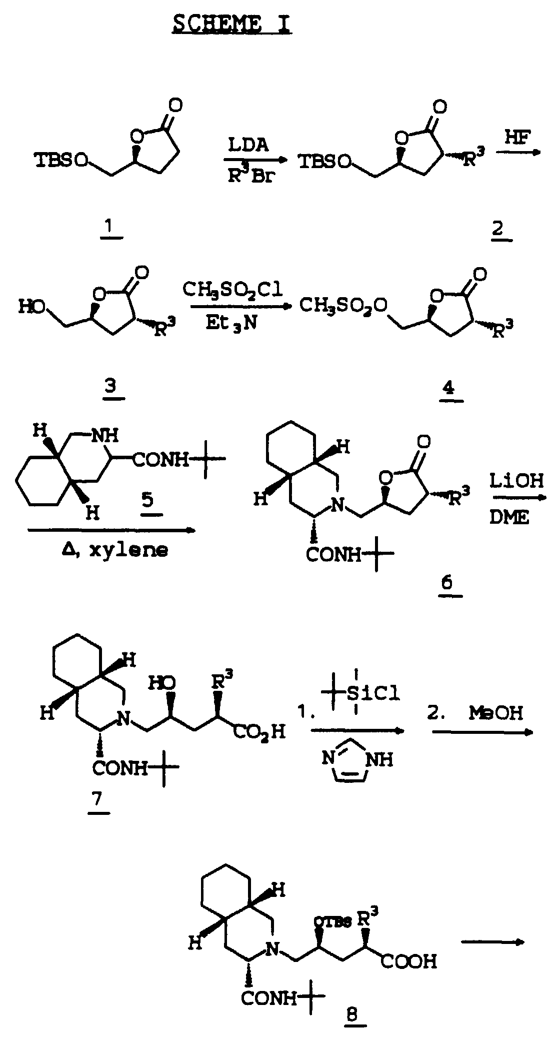

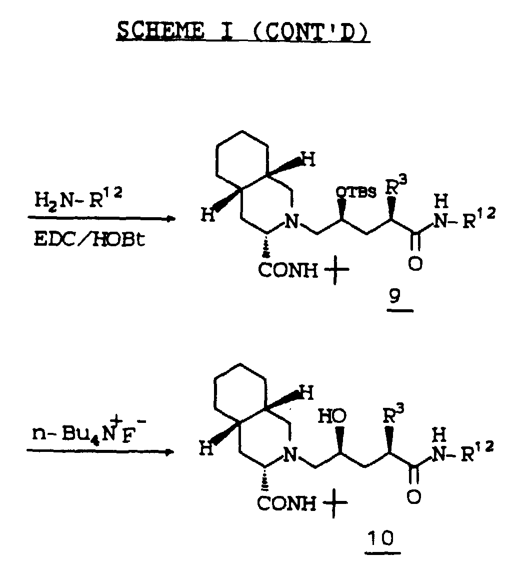

A second method for forming products of general formula I is shown in Scheme II. In Scheme II, alkylation of 11 is performed by a first step of deprotonation of 11 with n-butyllithium or lithium diisopropylamide (LDA) followed by a second step of adding an alkenyl halide (such as allyl bromide) to afford 12.

Dihydroxylation of the olefin of 12 with osmium tetroxide and N-methylmorpholine-N-oxide (NMO) produces a diasteriomeric mixture of diols, 13. Selective mesylation of the primary alcohol of 13 with methanesulfonyl chloride and either triethylamine or pyridine gives a mesylate 14.

Heating mesylate 14 with an amine in a refluxing alcoholic solvent such as methanol or isopropanol which contains an excess of potassium carbonate produces an amino alcohol such as compound 15. The diasteriomers can be separated at this step by standard techniques well known to those of skill in the art. Alternatively, the separation can be done after removal of the ketal.

Removal of the ketal in compound 15 is accomplished by treatment with acid in the presence of methanol, or by aqueous acid or by 1N HCl in THF, to form compound 16.

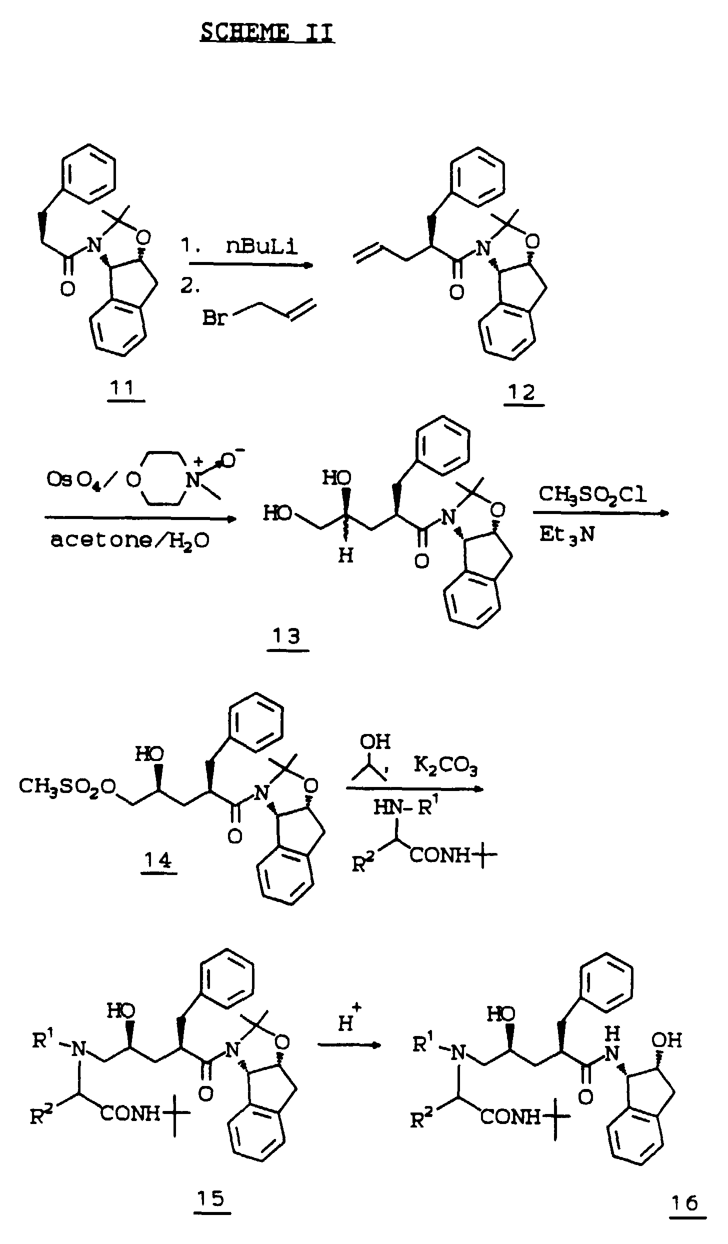

A third method for forming products of general formula I is shown in Scheme III. Protection of the pyrrolidine -NH- group of compound 17 is carried out with BOC-anhydride and dimethylaminopyridine to give the protected compound 18. Alkylation of 18 is performed by a first step of deprotonation of 18 with a strong base such as lithium hexamethyldisilamide (LHMDS) or lithium diisopropylamide (LDA) followed by a second step of adding an alkyl halide (such as benzyl bromide) to afford compound 19.

The TBS protecting and BOC protecting group of 19 are removed by treatment with aqueous HF in acetonitrile to give alcohol 20. Mesylation of the primary alcohol of 20 with methanesulfonyl chloride and either triethylamine or pyridine gives mesylate 21 which is heated with an amine in a refluxing alcoholic solvent such as methanol or isopropanol which contains an excess of potassium carbonate to produce an amino pyrrolidinone such as compound 22. The pyrrolidine -NH- group of 22 is reprotected as a BOC group as before and the resultant compound 23 is hydrolized open with a base such as lithium or sodium hydroxide to afford the acid 24. Compound 24 is then coupled to an NH₂R¹² amine in a standard manner and the BOC is removed with gaseous HCl or trifluoroacetic acid to give the desired product, exemplified by compound 25.

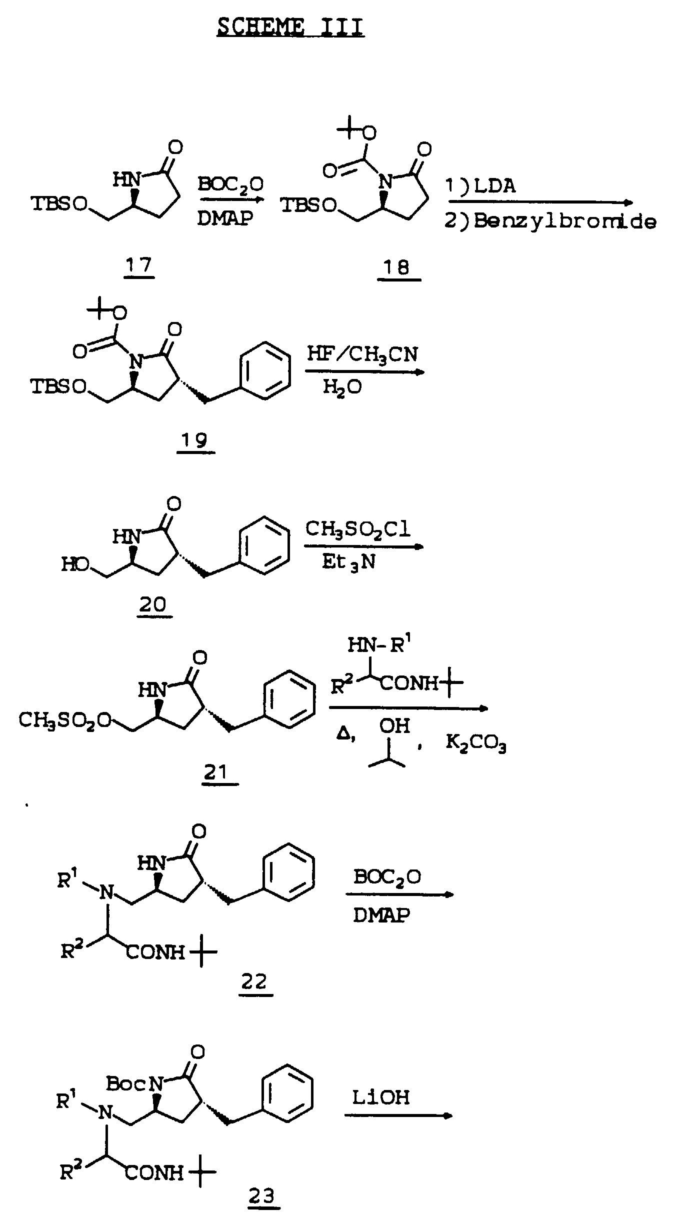

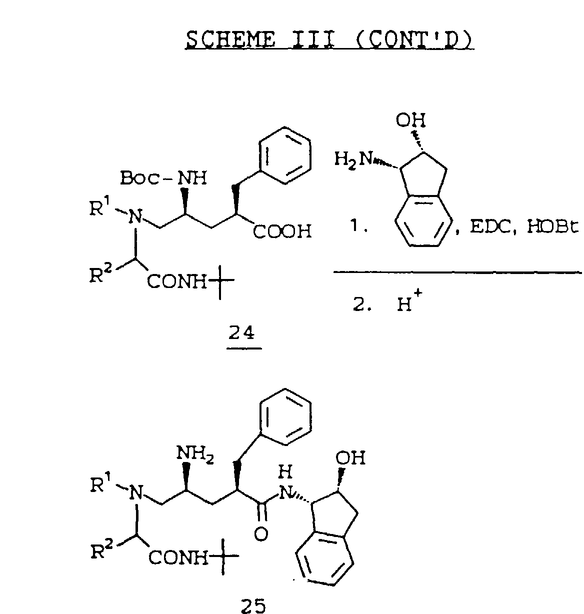

A compound of formula 26 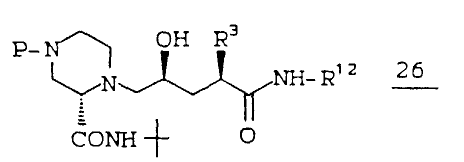 wherein P is a nitrogen protecting group such as -BOC or -CBZ, is preferably prepared according to the method described in Scheme I, preferably employing the 5-trifluoromethanesulfonyloxymethyl analog of lactone 4 therein (see Example 15, Step 1).

Compounds of formula 27 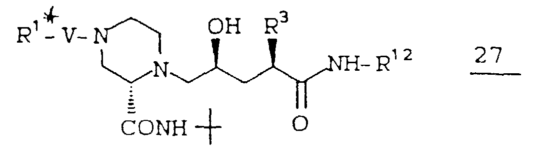 can be obtained by a variety of routes from compound 28 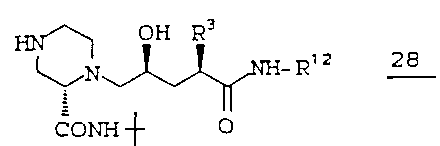 which is obtained after removal of the nitrogen protecting group in 26 using methods well known in the art, e.g., catalytic hydrogenation to remove a CBZ group, or treatment with trimethylsilyltriflate and 2,6 lutidine at about 0°C in a solvent such as CH₂Cl₂ to remove a BOC group.

For example, the 4-position piperazinyl nitrogen of compound 28 can be alkylated with a compound of formula R¹*-X in a solvent such as DMF in the presence of Et₃N at room temperature, wherein X is -Cl, Br or -I, or a sulfonamide group can be formed by treatment of 28 with a sulfonyl chloride compound of formula R¹*SO₂Cl under similar conditions. Also, standard amide coupling techniques can be used to form an amide group at the piperazinyl 4-position. Techniques for these procedures are well known to those skilled in the art. The R¹* group of R¹*-X or R¹*SO₂Cl is defined above in the definition of compounds of formula I except that R¹* can not be hydrogen or a group with a free hydroxy substituent, such as -C₁₋₄alkyl substitued with hydroxy, with the further exception that R¹* can be aryl substituted with a hydroxy group.

The compounds of this invention are also illustrated by Tables I-IV, which follow.

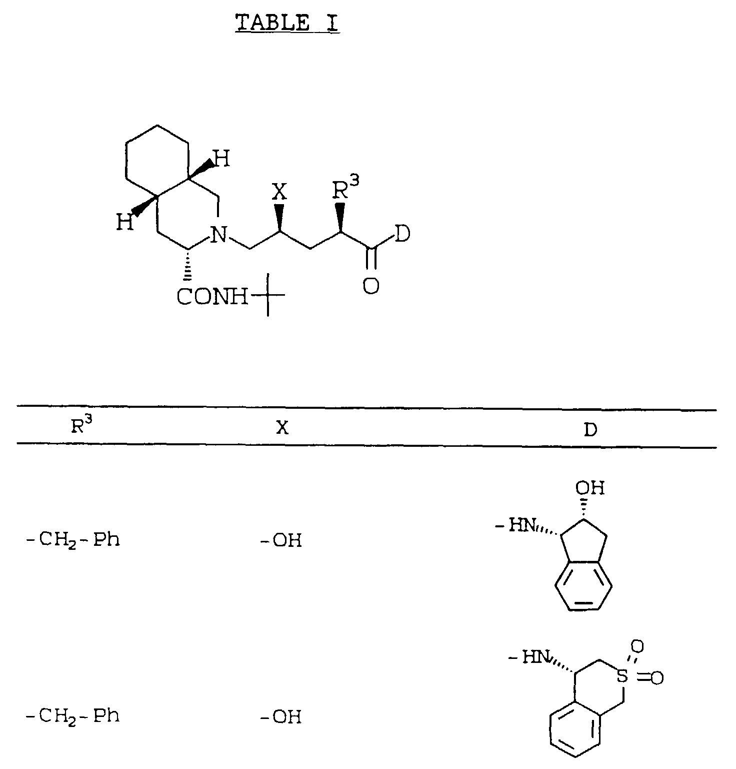

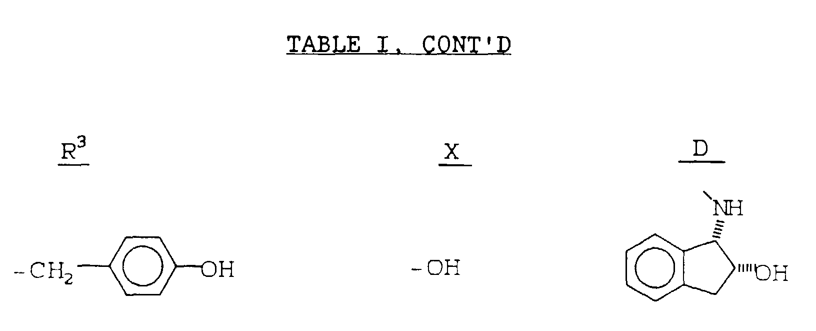

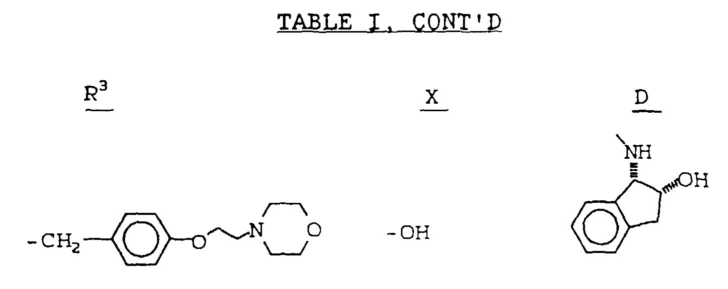

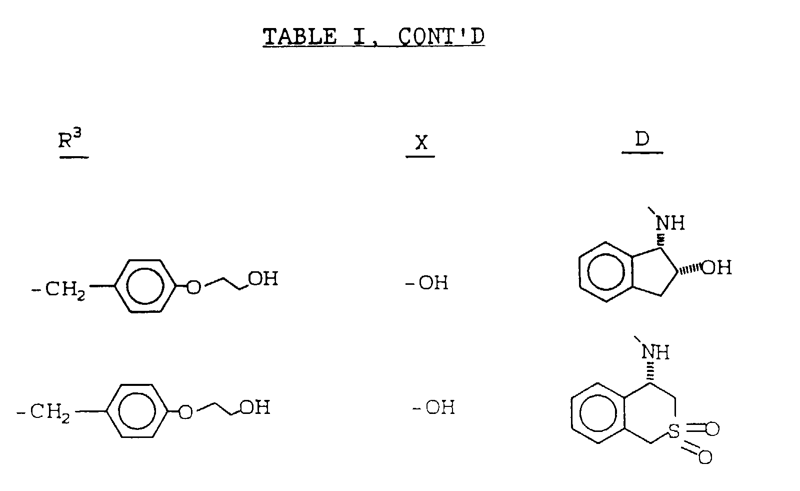

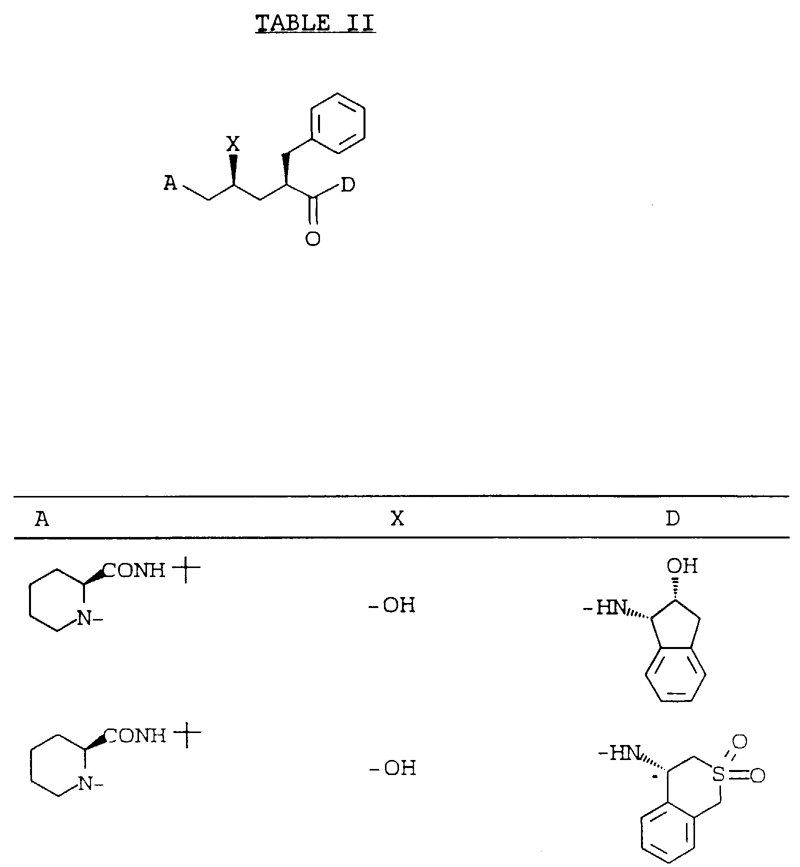

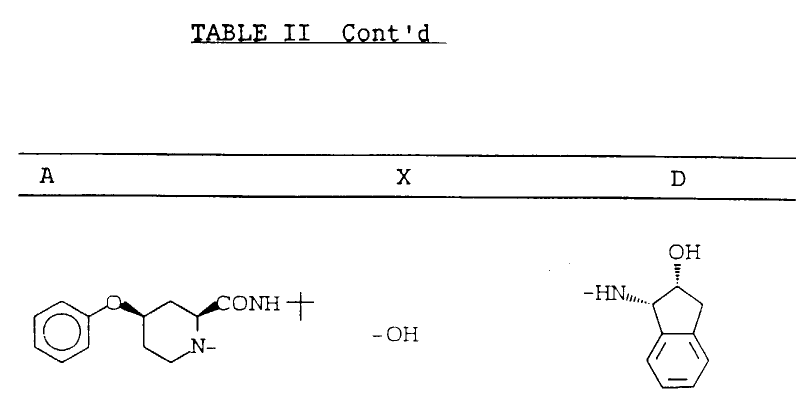

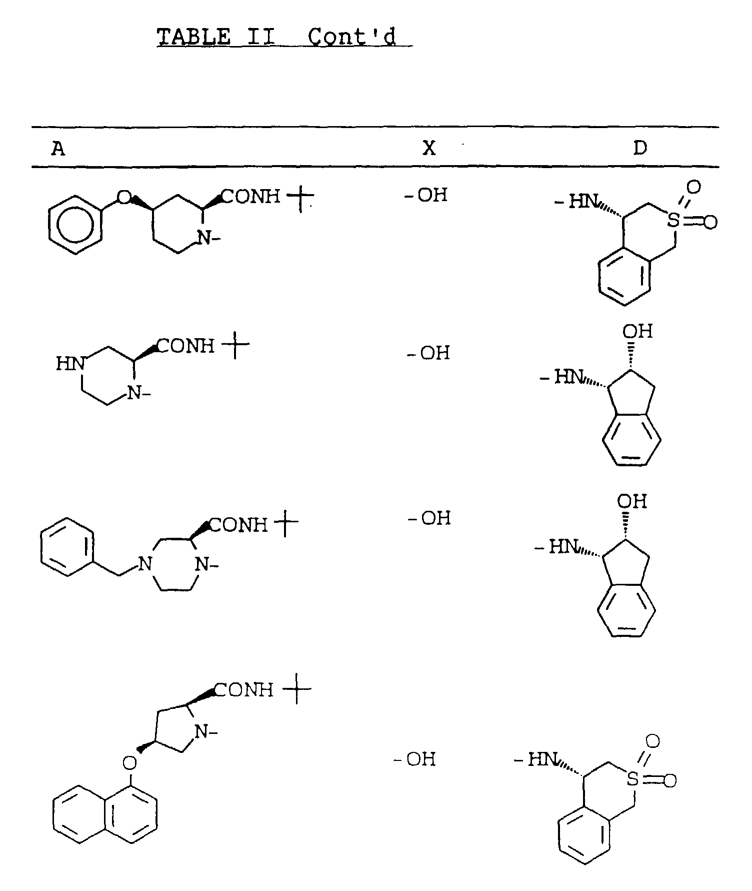

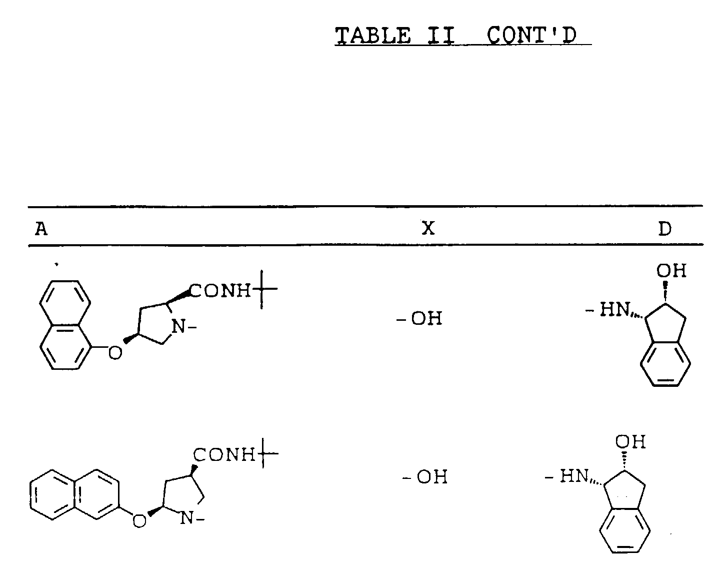

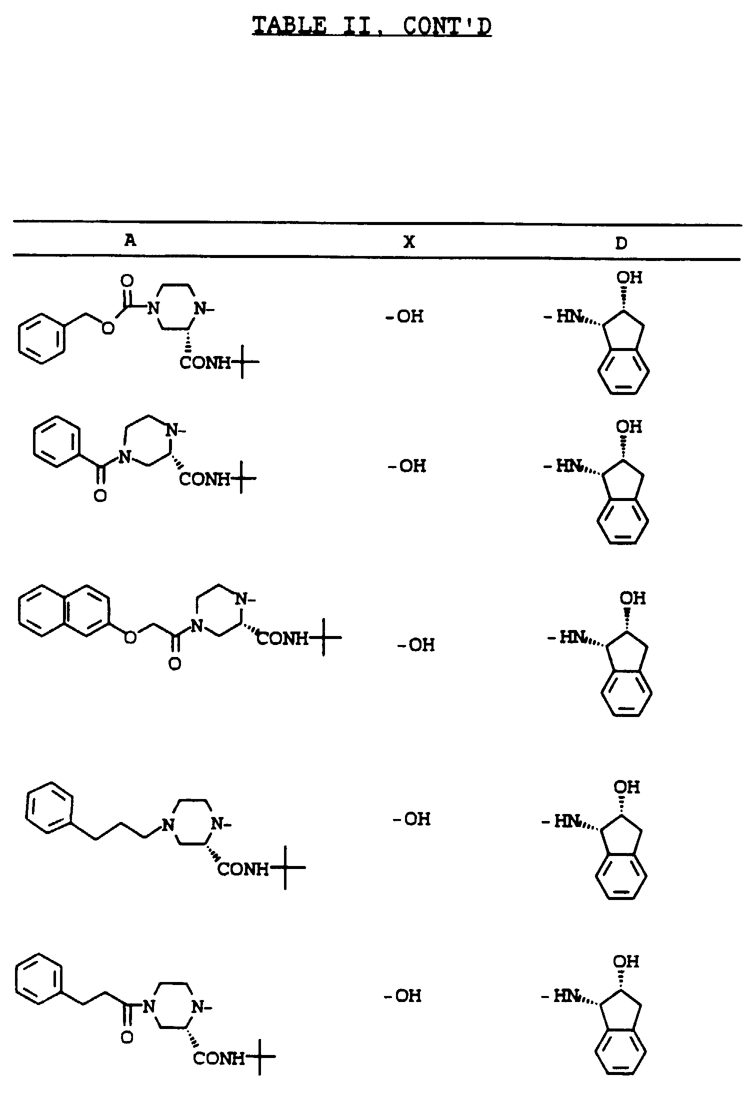

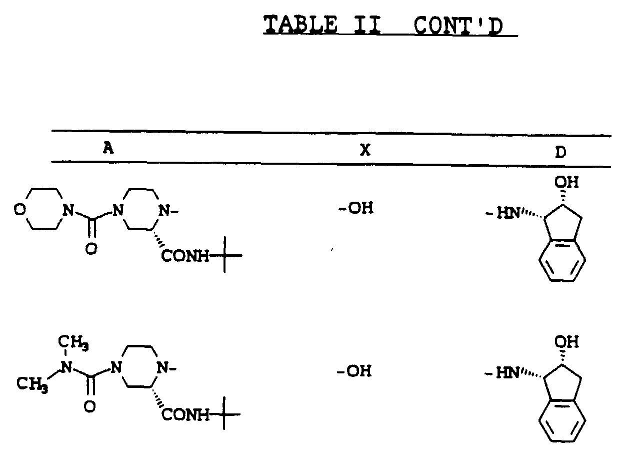

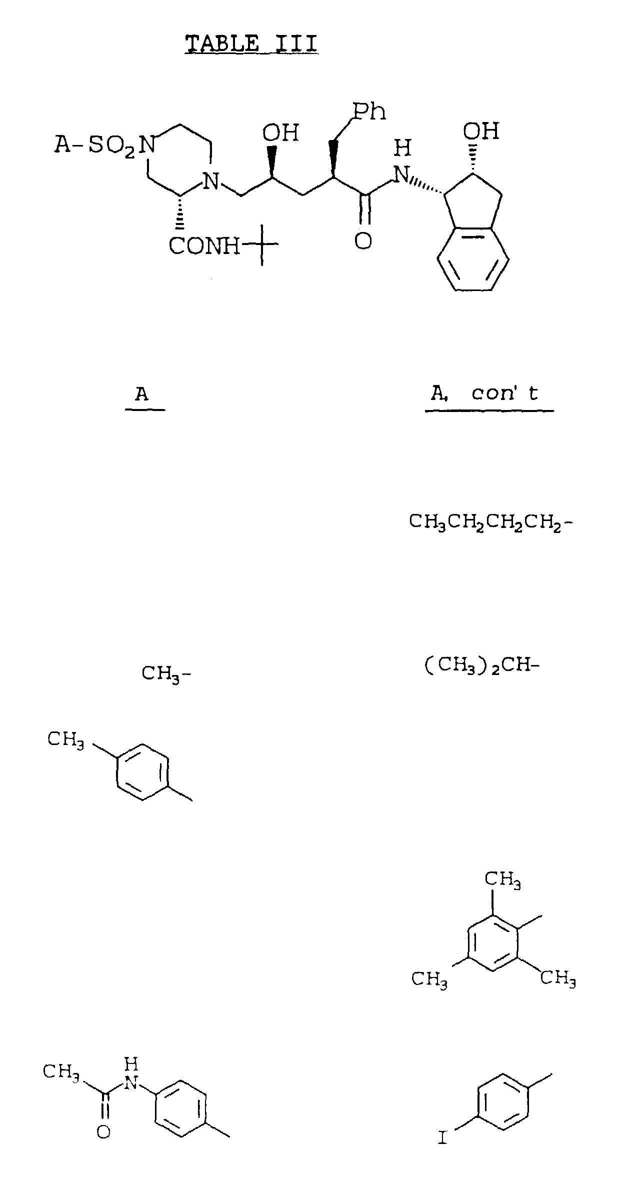

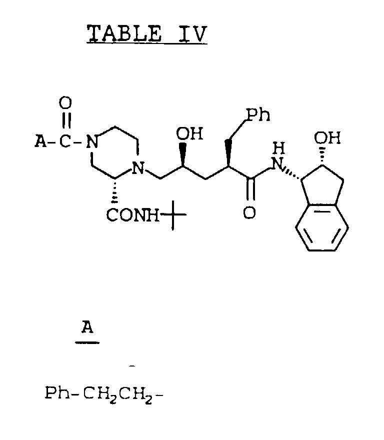

The compounds of the present invention are useful in the inhibition of HIV protease the prevention or treatment of infection by the human immunodeficiency virus (HIV) and the treatment of consequent pathological conditions such as AIDS. Treating AIDS or preventing or treating infection by HIV is defined as including, but not limited to, treating a wide range of states of HIV infection: AIDS, ARC (AIDS related complex), both symptomatic and asymptomatic, and actual or potential exposure to HIV. For example, the compounds of this invention are useful in treating infection by HIV after suspected past exposure to HIV by, e.g., blood transfusion, organ transplant, exchange of body fluids, bites, accidental needle stick, or exposure to patient blood during surgery.

For these purposes, the compounds of the present invention may be administered orally, parenterally (including subcutaneous injections, intravenous, intramuscular, intrasternal injection or infusion techniques), by inhalation spray, or rectally, in dosage unit formulations containing conventional non-toxic pharmaceutically-acceptable carriers, adjuvants and vehicles.

Thus, in accordance with the present invention there is further provided a method of treating and a pharmaceutical composition for treating HIV infection and AIDS. The treatment involves administering to a patient in need of such treatment a pharmaceutical composition comprising a pharmaceutical carrier and a therapeutically effective amount of a compound of the present invention, or a pharmaceutically acceptable salt thereof.

These pharmaceutical compositions may be in the form of orally-administrable suspensions or tablets; nasal sprays; sterile injectable preparations, for example, as sterile injectable aqueous or oleagenous suspensions or suppositories.

When administered orally as a suspension, these compositions are prepared according to techniques well-known in the art of pharmaceutical formulation and may contain microcrystalline cellulose for imparting bulk, alginic acid or sodium alginate as a suspending agent, methylcellulose as a viscosity enhancer, and sweetners/flavoring agents known in the art. As immediate release tablets, these compositions may contain microcrystalline cellulose, dicalcium phosphate, starch, magnesium stearate and lactose and/or other excipients, binders, extenders, disintegrants, diluents and lubricants known in the art.

When administered by nasal aerosol or inhalation, these compositions are prepared according to techniques well-known in the art of pharmaceutical formulation and may be prepared as solutions in saline, employing benzyl alcohol or other suitable preservatives, absorption promoters to enhance bioavailability, fluorocarbons, and/or other solubilizing or dispersing agents known in the art.

The injectable solutions or suspensions may be formulated according to known art, using suitable non-toxic, parenterally-acceptable diluents or solvents, such as mannitol, 1,3-butanediol, water, Ringer's solution or isotonic sodium chloride solution, or suitable dispersing or wetting and suspending agents, such as sterile, bland, fixed oils, including synthetic mono- or diglycerides, and fatty acids, including oleic acid.

When rectally administered in the form of suppositories, these compositions may be prepared by mixing the drug with a suitable non-irritating excipient, such as cocoa butter, synthetic glyceride esters or polyethylene glycols, which are solid at ordinary temperatures, but liquidify and/or dissolve in the rectal cavity to release the drug.

Dosage levels of the order of 0.02 to 5.0 or 10.0 grams-per-day are useful in the treatment or prevention of the above-indicated conditions, with oral doses two-to-five times higher. For example, infection by HIV is effectively treated by the administration of from 10 to 50 milligrams of the compound per kilogram of body weight from one to three times per day. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The present invention is also directed to combinations of the HIV protease inhibitory compounds with one or more agents useful in the treatment of AIDS. For example, the compounds of this invention may be effectively administered, whether at periods of pre-exposure and/or post-exposure, in combination with effective amounts of the AIDS antivirals, immunomodulators, anti-infectives, or vaccines known to those of ordinary skill in the art.

It will be understood that the scope of combinations of the compounds of this invention with AIDS antivirals, immunomodulators, anti-infectives or vaccines include in principle any combination with any pharmaceutical composition useful for the treatment of AIDS.

### Assay for Inhibition of Microbial Expressed Viral Protease

Inhibition studies of the reaction of the protease expressed in Eschericia coli with a peptide substrate [Val-Ser-Gln-Asn-(betanapthyl)Ala-Pro-Ile-Val, 0.5 mg/mL at the time the reaction is initiated] were in 50 mM Na acetate, pH 5.5, at 30°C for 1 hour. Various concentrations of inhibitor in 1.0 ul DMSO were added to 25 ul of the peptide solution in water. The reaction is initiated by the addition of 15 ul of 0.33 nM protease (0.11 ng) in a solution of 0.133 M Na acetate pH 5.5 and 0.1% bovine serum albumin. The reaction was quenched with 160 ul of 5% phosphoric acid. Products of the reaction were separated by HPLC (VYDAC wide pore 5 cm C-18 reverse phase, acetonitrile gradient, 0.1% phosphoric acid). The extent of inhibition of the reaction was determined from the peak heights of the products. HPLC of the products, independently synthesized, proved quantitation standards and confirmation of the product composition. The products of synthesis in Examples 1-7 inclusive showed IC₅₀ values in the range of 1-100 nM. Compounds A, B and J showed IC₅₀ values of between about 0.3 and about 6 nM.

### EXAMPLE 1

### Preparation of N-(2(R)-hydroxy-1(S)-indanyl)-2(R)-phenylmethyl-4(S)-hydroxy-5-(1-(N'-(t-butyl)-4(S)-phenozyprolineamide)yl)-pentaneamide

### Step 1: Preparation of N-(2(R)-hydroxy-1(S)-indanyl)3-phenylpropaneamide

To a cold (0°C) solution of methylene chloride (30 ml) containing 2(R)-hydroxy-1(S)-aminoindane (750 mg, 5.0 mmol) and triethylamine (606 mg, 6.0 mmol) was added a solution of hydrocinnamoyl chloride (843 mg, 5.0 mmol) in 5 ml of methylene chloride. After 2 hr the reaction was poured into a separatory funnel containing 50 ml of methylene chloride and washed with 10% citric acid solution (2 x 30 ml). The organic layer was dried, filtered and concentrated to afford a white solid.

### Step 2: Preparation of N-(2(R)-hydroxy-1(S)-indan-N,O-isopropylidene-yl)-3-phenyl-propaneamide

The crude white solid from step 1 above was dissolved in 50 ml of methylene chloride and 5 ml of dimethoxypropane was added followed by the addition of 100 mg of p-toluenesulfonic acid. The reaction was stirred at room temperature for 18 hr and then poured into a separatory funnel and washed with saturated NaHCO₃ solution (2 x 30 ml). The organic layer was dried, filtered and concentrated to afford an oil which was chromatographed (SiO₂, 40% EtOAc/Hexane) to give an oil which eventually crystallized.

### Step 3: Preparation of N-(2(R)-hydroxy-1(S)-indan-N,O-isopropylidene-yl)-2(S)-phenylmethylpent-4-eneamide

To a solution of N-(2(R)-hydroxy-1(S)-indan-N,O-isopropylidene-yl)-3-phenyl-propaneamide (1.03 gm, 2.9 mmol) in 20 ml of THF cooled to -78°C was added n-BuLi (2.5M, 1.40 ml, 3.5 mmol). After 20 min, allyl bromide (0.48 gm, 3.9 mmol) was added, the reaction was stirred at -78°C for 1 hr and then 10 ml of saturated NH₄Cl solution was added to quench the reaction. The reaction was diluted with 50 ml of water, extracted with ethyl acetate (2 x 50 ml), the organic phase was washed with saturated NaCl solution (50 ml), dried filtered and concentrated to afford the crude product. The crude product was purified on silica gel to afford the title compound.

### Step 4: Preparation of N-(2(R)-hydroxy-1(S)-indan-N,O-isopropylidene-yl)-2(S)-phenylmethyl-(4(RS),5-dihydroxy)-pentaneamide

To 800 mg (2.2 mmol) of N-(2(R)-hydroxy-1(S)-indan-N,O-isopropylidene-yl)-2(S)-phenylmethyl-pent-4-en eamide dissolved in 40 ml of a 9:1 mixture of acetone/water was added 0.8 ml of a 60% solution of N-methylmorpholine-N-oxide in water followed by 4 ml of a 2.5% solution of osmium tetroxide in t-BuOH. After 18 hr, excess solid sodium bisulfate was added, the reaction was stirred for 2 hr and then filtered through a pad of celite. The filtrate was concentrated, diluted with 50 ml of water, extracted with methylene chloride (2 X 50 ml), the organic phase was dried, filtered and concentrated to give the product as a foam.

### Step 5: Preparation of N-(2(R)-hydroxy-1(S)-indan-N,O-isopropylidene-yl)-2(S)-phenylmethyl-4(RS)-hydroxy-5-methanesulfonyloxypentaneamide

To 200 mg (0.527 mmol) of N-(2(R)-hydroxy-1(S)-indan-N,O-isopropylidene-yl)-2(S)-phenylmethyl-(4(RS),5-dihydroxy)-pentaneamide dissolved in 7 ml of methylene chloride at 0°C was added triethylamine (59 mg, 0.58 mmol), followed by methanesulfonyl chloride (66 mg, 0.579 mmol). After 4 hr the reaction was worked up by washing with 10% citric acid solution (2 X 50 ml) and the organic phase was dried, filtered and concentrated to afford the monomesylate as a mixture of alcohols.

### Step 6: Preparation of N'-t-butyl-N-Boc-4(R)-hydroxy-L-prolineamide

To a solution of N-Boc-4(R)-hydroxyproline (2.00 g) in DMF (20 mL) cooled to 0°C was added EDC (1.987 g), HOBt (1.401 g), tert butyl amine (1.09 mL) and triethylamine (2.41 mL). After 18 h the reaction mixture was diluted with ethyl acetate (150 mL) and washed with 10% HCl, saturated NaHCO₃, water and brine. The solution was then dried over MgSO₄ and concentrated to afford a white solid.

### Step 7: Preparation of N'-t-butyl-N-Boc-4(S)-phenoxy-L-prolineamide

To a solution of N'-t-butyl-N-Boc-4(R)-hydroxy-L-prolineamide (0.6 g) in THF (5 mL) was added phenol (0.295 g), triphenylphosphine (0.824 g) and then diethylazo-dicarboxylate (0.495 mL) dropwise. The reaction mixture stirred for 24 h at ambient temperature and was diluted with ethyl acetate (200 mL) and washed with saturated NaHCO₃, water, brine and dried over MgSO₄. Concentration in vacuo afforded a yellow oil which was purified by flash chromatography (elution hexane: EtOAc 1:1, 30 mm column).

### Step 8: Preparation of N-t-butyl-4(S)-phenoxy-L-prolineamide trifluoroacetic acid salt

To a solution of N'-t-butyl-N-Boc-4(S)-phenoxy-L-prolineamide (0.596 g) in methylene chloride (4 mL) at 0°C was added trifluoroacetic acid (2 mL). After 30 min the reaction was warmed to room temperature and stirred for two hours. The solvent was removed in vacuo and a slightly yellow oil was obtained.

### Step 9: Preparation of N-(2(R)-hydroxy-1(S)-indan-N,O-isopropylidene-yl)-2-(R)-phenylmethyl-4-(S)-hydroxy-5-(1-(N'-(t-butyl)-4(S)-phenoxyprolineamide)yl)-pentaneamide

To a solution of N-t-butyl-4(S)-phenoxy-L-prolineamide trifloroacetic acid salt (0.36 g) and N-(2(R)-hydroxy-1(S)-indan-N,O-isopropylidene-yl)-2(S)-phenylmethyl-4(RS)-hydroxy-5-methanesulfonyloxypentaneamide (0.226 g) in 3 mL of isopropanol was added potassium carbonate (0.441 g) and the reaction was warmed to 80°C. After 18 h the reaction was cooled to room temperature, filtered through celite which was washed with further portions of EtOAc. The filtrate was concentrated, the residue was dissolved in EtOAc (100mL) and washed with water, brine and dried over MgSO₄. The solvent was removed in vacuo and the resulting oil was purified by flash chromatography to afford the product as a mixture of diastereomers.

### Step 10: Prep of N-(2(R)-hydroxy-1(S)-indanyl)-2-(R)-phenylmethyl-4-(S)-hydroxy-5-(1-(N'-t-butyl-4(S)-phenoxyprolineamid)yl)-pentaneamide

To a solution of N-(2(R)-hydroxy-1(S)-indan-N,O-isopropylidene-yl)-2-(R)-phenylmethyl-4-(S)-hydroxy-5-(1-(N'-(t-butyl)-4(S)-phenoxyprolineamide)yl)-pentaneamide (0.13 g) in MeOH (5 mL) was added camphorsulfonic acid (CSA) (0.070 g) at ambient temperature. After 5 hours more CSA (0.025 g) was added and the reaction was stirred for total of 18 hours. The reaction was quenched with saturated NaHCO₃ (5 mL) and the solvent was removed to a volume of 4 mL. The aqueous layer was thoroughly extracted with EtOAc and the organic layer was washed with water, brine and dried. After removal of the solvent in vacuo the resulting oil was purified via flash chromatography to provide the title compound as a white foam. The foam was dissolved in EtOAc : hexanes and the mother liquor was decanted away from the oil. The oil was then dried in a high vacuum desiccator to afford a white foam.

### EXAMPLE 4

### Preparation of N-(2(R)-hydroxy-1(S)-indanyl)-2(R)-phenylmethyl-4(S)-hydroxy-5-(2-(3(S)-N'-(t-butylcarboxamido)-(4aS,8aS)-decahydroisoquinoline)yl)pentaneamide

### Step 1: Preparation of dihydro-5(S)-((t-butyldiphenylsilyl)oxymethyl)-3(R)phenylmethyl-3(2H)-furanone

A solution of lithium diisopropylamide (LDA) was generated by the addition 1.55 ml of n-BuLi (2.5M in hexane) to 0.55 ml (3.9 mmol) of diisopropylamine in 10 ml of THF at -78°C. After 30 minutes a solution of dihydro-5-(S)-((t-butyldiphenylsilyl)-oxymethyl)-3(2H)-furanone (1.38 g, 3.89 mmol) in 5 ml of THF was added. After an additional 30 minutes of stirring, benzyl bromide (0.68 g, 3.9 mmol) was added and stirring was continued for 3 h after which time the reaction was quenched with the addition of a 10% aqueous citric acid solution. The solution was extracted with ethyl acetate (2 x 50 ml) which was backwashed with brine, dried, filtered and concentrated to afford an oil. The product was purified by chromatography (SiO₂, 20% EtOAc/Hexane) to afford the title compound.

### Step 2: Preparation of dihydro-5(S)-(hydroxymethyl)-3 (R)-phenylmethyl-3(2H)-furanone

To 5.26 g of dihydro-5(S)-((t-butyldiphenylsilyl)oxymethyl)-3(R)phenylmethyl-3(2H)-furanone in 40 ml of acetonitrile was added 1.34 ml of a 49% aqueous HF solution. After 18 hr at room temperature the reaction was concentrated to dryness and the residue was partitioned between water (50 ml) and ethyl acetate (50 ml). The organic layer was washed with brine, dried filtered and concentrated to afford the product as a tan solid (mp 69-72°C).

### Step 3: Preparation of dihydro-5(S)-((methanesulfonyl)oxymethyl)-3(R)phenylmethyl-3(2H)-furanone

To a solution of 2.93 g (14 mmol) of dihydro-5(S)-(hydroxymethyl)-3(R)-phenylmethyl-3(2H)-fur anone in methylene chloride cooled to 0°C was added triethylamine (1.98ml, 15.6 mmol) followed by the addition of methanesulfonyl chloride (1.20 ml, 15.6 mmol). After 1 hour at 0°C, the reaction was poured into 10% aqueous citric acid solution, washed with ethyl acetate (2 x 100 ml) which was backwashed with water (100 ml), brine (100 ml), dried, filtered and concentrated to give the product as a waxy brown solid.

### Step 4: Preparation of dihydro-5(S)-(2-(3(S)-N-(t-butylcarboxamido)-(4aS, 8aS)-(decahydroisoquinoline)yl)methyl)-3(R)-phenylmethyl-3(2H)-furanone

To 70 mg of dihydro-5(S)-((methanesulfonyl)oxymethyl)-3(R)phenylmethyl-3(2H)-furanone (0.25 mmol) in 10 ml of xylene containing 100 mg of potassium carbonate was added 65 mg (0.27 mmol) of N-t-butyl-(4aS,8aS)-(decahydroisoquinoline)-3(S)-carboxamide and the reaction was heated to 140°C. After 6 hours, the reaction was cooled, poured into 30 ml of water which was washed with ethyl acetate (2 x 30 ml). The organic phase was dried, filtered and concentrated to afford a residue which was chromatographed (50/50 EtOAc/Hexane) to give the product.

### Step 5: Preparation of 2(R)-phenylmethyl-4(S)-(t-butyldimethylsilyloxy)-5-(2-(3(S)-N-(t-butylcarboxamido)-(4aS,8aS)-decahydroisoquinoline)yl)-pentanoic acid

To 130 mg (0.305 mmol) of dihydro-5(S)-(2-(3(S)-N-(t-butylcarboxamido)-(4aS, 8aS)-(decahydroisoquinoline)yl)methyl)-3(R)-phenylmethyl-3-(2H)furanone in 2 ml of DME was added 1 ml lithium hydroxide solution. After 4 hours at room temperature, the reaction was concentrated to dryness and azeotroped with toluene (3X) to remove excess water. The residue was dissolved in 5 ml of DMF and 414 mg (6.10 mmol) of imidazole and 465 mg (3.05 mmol) of t-butyldimethylsilyl chloride was added. After two days at room temperature, 1 ml of methanol was added to the reaction and after 1 hour the solution was evaporated to dryness. The residue was partitioned between saturated NH₄Cl solution (aq) and washed with ethyl acetate which was dried, filtered and concentrated to give an oil which was a mixture of product and the furanone starting material. This material was carried on crude into the next reaction.

### Step 6: Preparation of N-(2(R)-hydroxy-1(S)-indanyl)-2(R)-phenylmethyl-4(S)-(t-butyldimethylsilyloxy-5-(2-(3(S)-N'-(t-butylcarboxamido)-(4aS,8aS)-decahydroisoquinoline)yl)pentaneamide

The crude product of step 5, above, was dissolved in 3 ml of DMF along with 47 mg (0.246 mmol) of EDC, 33 mg (0.246 mmol) of HOBT and 37 mg of 2(R)-hydroxy-1(S)-aminoindane. The pH of the solution was adjusted to 8.5-9.0 with triethylamine and after 18 hours it was worked up by concentrating to dryness, dissolving the residue in 10% aq. citric acid solution and washing the aqueous layer with ethyl acetate. The organic layer was dried, filtered and concentrated and the resultant oil was chromatographed (SiO₂, 30% EtOAc/Hexane) to yield the title compound.

### Step 7: Preparation of N-(2(R)-hydroxy-1(S)-indanyl)-2(R)-phenylmethyl-4(S)-hydroxy-5-(2-(3(S)-N'-(t-butylcarboxamido)-(4aS,8aS)-decahydroisoquinoline)yl)-pentaneamide

The product from step 6, above, was dissolved in 1 ml of THF and 1 ml of a 1M solution of tetrabutylammonium fluoride in THF was added. After 18 hr at room temperature the reaction was diluted with 20 ml of saturated NaHCO₃ solution (aq) and the product was extracted into ethyl acetate which was dried, filtered and concentrated to give a foam. The resultant material was chromatographed on a prep plate (0.5 mm, 5% MeOH/CECl₃) and the title product isolated in the usual manner as a solid with mp 105-107°C.

### EXAMPLE 6

### Preparation of N-(2(R)-hydroxy-1(S)-indanyl)-2(R)-phenylmethyl-4(S)-hydroxy-5-(1-(4-carbobenzyloxy-2-(S)-N'-(t-butylcarboxamido)-piperazinyl))-pentaneamide

Employing substantially the same procedure used in Example 1, but substituting N-t-butyl-4-CBZ-piperazine-2(S)-carboxamide for N-t-butyl-4(S)-phenoxy-L-prolineamide used in step 9 therein, the title compound was obtained.

### EXAMPLE 9

### Preparation of N-(4(S)-3,4-dihydro-1H-2,2-dioxobenzothiopyranyl)-2(R)-phenylmethyl-4(S)-hydroxy-5-(2-(3(S)-N'-(t-butylcarboxamido)-(4aS,8aS)-decahydroisoquinoline)yl-pentaneamide

### Step 1: Preparation of N-(4(S)-3,4-dihydro-1H-benzothiopyranyl)-2(R)-phenylmethyl-4(S)-hydroxy-5-(2-(3(S)-t-butylcarboxamido)-(4aS, 8aS)-decahydroisoquinoline)yl)-pentaneamide

Employing substantially the same procedure used in Example 4 but substituting 4(S)-amino-3,4-dihydro-1H-benzothiopyran for the 2(R)-hydroxy-1(S)-aminoindane used in step 6 therein, the title compound is obtained.

### Step 2: Preparation of N-(4(S)-3,4-dihydro-1H-2,2-dioxobenzothiopyranyl)-2(R)-phenylmethyl-4(S)-hydroxy-5-(2-(3(S)-t-butylcarboxamido)-(4aS,8aS)-decahydroisoquinoline)yl)-pentaneamide

The compound from step 1 above is dissolved in a 1:1 mixture of methanol and water. To this is added 10 eq. of OXONE and the reaction is stirred at room temperature. When the reaction is complete, it is concentrated to dryness, water is added and extracted with ethyl acetate which is dried, filtered and concentrated to give the title compound.

### EXAMPLE 10

### Preparation of N-(4(S)-3,4-dihydro-1H-2,2-dioxobenzothiopyranyl)-2(R)-phenylmethyl-4(S)-hydroxy-5-(1-(4-carbobenzyloxy-2(S)-N'-(t-butylcarboxamido)piperazinyl))-pentaneamide

### Step 1: Preparation of dihydro-5(S)-(1-(4-carbobenzyloxy-2(S)-N'-(t-butylcarboxamido)piperazinyl)methyl)-3(R)-phenylmethyl-3(2H)-furanone

Employing substantially the same procedure used in Example 4, step 4 but substituting 4-carbobenzyloxy-2(S)-N'-(t-butylcarboxamido)-piperazine for the N'-t-butyl-(4aS,8aS)-(decahydroisoquinoline)-3(S)-carboxamide used therein, the title compound is produced.

### Step 2: Preparation of 2(R)-phenylmethyl-4(S)-(t-butyldimethylsilyloxy)-5-(1-(4-carbobenzyloxy-2(S)-N'-(t-butylcarboxamido)piperazinyl))-pentanoic acid

Employing substantially the same procedure used in Example 4, step 5 but substituting dihydro-5(S)-(1-(4-carbobenzyloxy-2(S)-N'-(t-butylcarboxamido)-piperazinyl)methyl)-3(R)-phenylmethyl-3(2H)-furanone for the dihydro-5(S)-(2-(3(S)-N'-(t-butylcarboxamido)-(4aS,8aS)-(decahydroisoquinoline)yl)methyl)-3(R)-phenylmethyl-3(2H) furanone used therein, the title compound is produced.

### Step 3: Preparation of N-(4(S)-3,4-dihydro-1H-benzothiopyranyl)-2(R)-phenylmethyl-4(S)-(t-butyldimethylsilyloxy)-5-(1-(4-carbobenzyloxy-2(S)-N'-(t-butylcarboxamido)piperazinyl))-pentaneamide

The crude 2(R)-phenylmethyl-4(S)-(t-butyldimethylsilyloxy)-5-(1-(4-carbobenzyloxy-2(S)-N'-(t-butylcarboxamido)-piperazinyl))-pentanoic acid is dissolved in 3ml of DMF along with 1 eq of EDC, 1 eq of HOBT and 1 eq of 4(S)-amino-3,4-dihydro-1H- benzothiop yran. The pH of the solution is adjusted to 8.5-9.0 with triethylamine and after 18 hours it is worked up by concentrating to dryness, dissolving the residue in 10% aq citric acid solution and washing the aqueous layer with ethyl acetate. The organic layer is dried, filtered and concentrated and the resultant residue is chromatographed to yield the title product.

### Step 4: Preparation of N-(4(S)-3,4-dihydro-1H-benzothiopyranyl)-2(R)-phenylmethyl-4(S)-hydroxy)-5-(1-(4-carbobenzyloxy-2(S)-(t-butylcarboxamido)-piperazinyl))-pentaneamide

The product from step 3 above is dissolved in 1 ml of THF and 1 ml of a 1M solution of tetrabutylammonium fluoride in THF is added. After 18 hr at room temperature the reaction is diluted with 20 ml of saturated NaHCO₃ solution (aq) and the product is extracted into ethyl acetate which is dried, filtered and concentrated to give a residue. The residue is chromatographed to afford the product.

### Step 5: Preparation of N-(4(S)-3,4-dihydro-1H-2,2-dioxobenzothiopyranyl)-2(R)-phenylmethyl-4(S)-hydroxy-5-(1-(4-carbobenzyloxy-2(S)-N'-(t-butylcarboxamido)-piperazinyl))-pentaneamide

The compound from step 4 above is dissolved in a 1:1 mixture of methanol and water. To this is added 10 eq of OXONE and the reaction is stirred at room temperature. When the reaction is complete, it is concentrated to dryness, water is added and extracted with ethyl acetate which is dried, filtered and concentrated to give the title compound.

### EXAMPLE 11

### Preparation of N-(2(R)-hydroxy-1(S)-indanyl)-2(R)-((4-((2-hydroxy)ethoxy)phenyl)methyl)-4(S)-hydroxy-5-(2-(3-(S)-N'-(t-butylcarboxamido)-(4aS,8aS)-decahydroisoquinoline)yl)pentaneamide

### Step 1: Preparation of N-(2(R)-hydroxy-1(S)-indanyl)-2(R)-((4-(2-allyloxy)phenyl)methyl)-4(S)-hydroxy-5-(2-(3(S)-t-butylcarboxamido)-(4aS,8aS)-decahydroisoquinoline)yl)pentaneamide

To a solution of N-(2(R)-hydroxy-1(S)-indanyl)-2(R)-((4-hydroxyphenyl)methyl)-4(S)-hydroxy-5-(2-(3(S)-t-butylcarboxamido)-(4aS,8aS)-decahydroisoquinoline)yl)-pentaneamide in dioxane is added 6 eq of allyl bromide and 6 eq of cesium carbonate. The reaction is heated to 90°C. When the reaction is complete, the precipitate is filtered off, the dioxane is concentrated to dryness and the residue is diluted with water which is washed with ethyl acetate. The organic phase is dried, filtered and concentrated to afford the product.

### Step 2: Preparation of N-(2(R)-hydroxy-1(S)-indanyl)-2(R)-((4-((2-hydroxy)ethoxy)phenyl)methyl)-4(S)-hydroxy-5-(2-(3(S)-N'-(t-butylcarboxamido)-(4aS,8aS)-decahydroisoquinoline)yl)pentaneamide

The product from step 1 above is dissolved in methanol, 1 eq of p-toluenesulfonic acid is added and the reaction is cooled to -78°C. Excess ozone is bubbled through the reaction until a blue color persists. The flask is purged with nitrogen to remove any ozone and excess sodium borohydride solution is added. The reaction is warmed to room temperature and then saturated NaHCO₃ solution is added. The methanol is concentrated off on the rotoevaporater and the aqueous residue is washed with ethyl acetate which is dried, filtered and concentrated to afford the title compound.

### EXAMPLE 12

### Preparation of N-(2(R)-hydroxy-1(S)-indanyl)-2(R)-((4-((2-hydroxy)ethoxy)phenyl)methyl)-4(S)-hydroxy-5-(1-(4-carbobenzyloxy-2(S)-N'-(t-butylcarboxamido)piperaziny))-pentaneamide

Employing substantially the same prodecure used in Example 11 but substituting N-(2(R)-hydroxy-1(S)-indanyl)-2(R)-((4-hydroxyphenyl)methyl)-4(S)-hydroxy-5-(1-(4-carbobenzyloxy-2(S)-(t-butylcarboxamido)-piperazinyl)-pentaneamide for the N-(2(R)-hydroxy-l(S)-indanyl)-2(R)-((4-hydroxyphenyl)methyl)-4(S)-hydroxy-5-(2-(3(S)-t-butylcarboxamido)-(4aS,8aS)-decahydroisoquinoline)yl)-pentaneamide used therein, the title compound is obtained.

### EXAMPLE 13

### Preparation of N-(2(R)-hydroxy-1(S)-indanyl)-2(R)-((4-(2-(4-morpholinyl)ethoxy)phenyl)methyl)-4(S)-hydroxy-5-(2-(3(S)-N'-(t-butylcarboxamido)-(4aS,8aS)-decahydroisoquinoline)yl)-pentaneamide

To a solution of N-(2(R)-hydroxy-1(S)-indanyl)-2(R)-((4-hydroxyphenyl)methyl)-4(S)-hydroxy-5-(2-(3(S)-N'-(t-butylcarboxamido)-(4aS,8aS)-decahydroisoquinoline)yl)-pentaneamide in dioxane is added 6 eq of chloroethyl morpholine and 6 eq of cesium carbonate. The reaction is heated to 90°C. When the reaction is complete, the precipitate is filtered off, the dioxane is concentrated to dryness and the residue is diluted with water which is washed with ethyl acetate. The organic phase is dried, filtered and concentrated to afford the title compound.

### EXAMPLE 14

### Preparation of N-(2(R)-hydroxy-1(S)-indanyl)-2(R)-((4-(2-(4-morpholinyl)ethoxy)phenyl)methyl)-4(S)-hydroxy-5-(1-(4-carbobenzyloxy-2(S)-N'-(t-butylcarboxamido)-piperazinyl))-pentaneamide

To a solution of N-(2(R)-hydroxy-1(S)-indanyl)-2(R)-((4-hydroxyphenyl)methyl)-4(S)-hydroxy-5-(1-(4-carbobenzyloxy-2(S)-(t-butylcarboxamido)piperazinyl)-pentaneamide in dioxane is added 6 eq of chloroethyl morpholine and 6 eq of cesium carbonate. The reaction is heated to 90°C. When the reaction is complete, the precipitate is filtered off, the dioxane is concentrated to dryness and the residue is diluted with water which is washed with ethyl acetate. The organic phase is dried, filtered and concentrated to afford the title compound.

### EXAMPLE 15

### Preparation of N-(2(R)-hydroxy-1(S)-indanyl)-2(R)-phenylmethyl-4(S)-hydroxy-5-(1-(4-(3-pyridylmethyl)-2(S)-N'-(t-butylcarboxamido)-piperazinyl))-pentaneamide

### Step 1: Preparation of dihydro-5(S)-((trifluoromethanesulfonyl)oxymethyl)-3(R)-phenylmethyl-3(2H)-furanone

To a solution of 18.4g (89.2 mmol) of dihydro-5(S)-(hydroxymethyl)-3(R)-phenylmethyl-3(2H)-furanone in 350 mL of methylene chloride cooled to 0°C was added 13.51 mL 2,6-lutidine (115.98 mmol) followed by a dropwise addition of 16.51 mL of trifluoromethanesulfonic anhydride (98.1 mmol). After 1.5 hours at 0°C, the reaction was poured into a mixture of 300 mL ice/brine and stirred for 0.5 hours. The aqueous layer was then extracted with methylene chloride (3 x 150 mL), the organic layers were washed with 10% HCl (2 x 75 mL), saturated NaHCO₃ (100mL), water (100mL), dried over MgSO₄, filtered and concentrated to give a solid residue. Purification via flash column chromatography (120 x 150 mm column, gradient elution of hexanes:EtOAc, 4:1 to 3:1) afforded the title product; mp 53-54°C.

### Step 2: Preparation of 4-(1,1-dimethylethyl)-1-(phenylmethyl)-1,2(S),4-piperazinetricarboxylate

The title compound was prepared following the procedure of Bigge, C.F.; Hays, S.J.; Novak, P.M.; Drummond, J.T.; Johnson, G.; Bobovski, T.P. Tetrahedron Lett. 1989, 30, 5193; starting with 2(S)-piperazinecarboxylic acid. (see Felder, E.; Maffei, S.; Pietra, S.; Pitre, D.; Helv. Chim. Acta 1960, 117, 888.

### Step 3: Preparation of N-t-butyl-4-(1,1-dimethylethoxycarbonylamino)-1-(phenylmethylcarbonylamino)piperazine-2(S)-carboxamide

To 9.90g (27.16 mmol) of 4-(1,1-dimethylethyl)-1-(phenylmethyl)-1,2(S),4-piperazinetricarboxylate dissolved in 75 mL of DMF and cooled to 0°C was added 5.73g (29.88 mmol) of EDC, 4.03g (29.88 mmol) of HOBt, 3.14 mL (29.88 mmol) of t-butylamine, and finally 4.16 mL (29.88 mmol) of triethylamine. The reaction mixture was stirred for 18 hours and the reaction volume was concentrated by half. The mixture was then diluted with 600 mL of EtOAc and washed with 10% HCl (2 x 75 mL), saturated NaHCO₃ (1 x 75 mL), water (3 x 75 mL) and brine (1 x 50 mL), dried over MgSO₄ and concentrated to a solid. This solid was triturated with EtOAc: hexane (1:2) and filtered to provide the title product as a white solid;
mp 134-135°C.

### Step 4: Preparation of N-t-butyl-4-(1,1-dimethylethoxycarbonylamino)piperazine-2(S)carboxamide

To 1.20g (2.86 mmol) of N-t-butyl-4-(1,1-dimethylethoxycarbonylamino)-1-(phenylmethylcarbonylamino)piperazine-2(S)-carboxamide and 1.1g (0.086 mmol) of 10% Pd/C was added 15 mL of methanol. The vessel was charged with hydrogen and the reaction stirred for 2 hours, filtered through celite and washed with ethanol. The solvents were removed in vacuo to provide the title product as a foam.
¹H NMR (300 MHz, CDCl₃) δ 6.65 (br, 1H), 4.10 (m, 1H), 3.81 (br, 1H), 3.21 (dd, J=18 and 7 Hz, 1H), 3.02-2.70 (m, 4H), 2.10-2.0 (br, 1H), 1.50 (s, 9H), 1.41(s, 9H).

### Step 5: Preparation of dihydro-5(S)-(4-(1,1-dimethylethoxycarbonylamino))-2(S)-N-(t-butylcarboxamido)-piperazinyl)methyl)-3(R)-phenylmethyl-3(2H)-furanone

To a solution of 22.40g (0.0662 mol) dihydro-5(S)-((trifluoromethanesulfonyl)oxymethyl)-3(R)-phenylmethyl-3(2H)-furanone (prep in step 1) and 18.0g (0.063mol) of n-t-butyl-4-(1,1-dimethylethoxycarbonylamino)piperazine-2(S)-carboxamide dissolved in 180 mL of isopropanol was added 11.53 mL (0.0662 mol) of N,N-diisopropylethylamine. After 2.5 hours another 1.2g of dihydro-5(S)-((trifluoromethanesulfonyl)oxymethyl)-3(R)-phenylmethyl-3(2H)-furanone was added. The reaction was complete by thin layer chromatography (t1c) after 3.5 hours and was concentrated to a thick oil. Trituration with EtOAc:hexanes (1:2, 200mL) provided a white solid which was filtered and discarded. The oil was purified by flash column chromatography (120 x 150 mm column, EtOAc:hexanes gradient elution 1:1, 2:1, 3:1 to all EtOAc) to afford the title compound.
¹H NMR (400 MHz, CDCl₃) δ 7.34-7.17 (m, 5H), 6.31 (br s, 1H), 4.38 (br m, 1H), 3.96-3.92 (m, 1H), 3.79 (br m, 1H), 3.16 (dd, J=13.6 and 4.4 Hz, 1H), 3.08-2.99 (m, 3H), 2.90-2.82 (m, 1H), 2.80 (dd, J=13.5 and 8.9 Hz, 1H), 2.78 (m, 1H), 2.67-2.61 (m,1H), 2.58-2.49 (m, 1H), 2.38-2.32 (m,1H), 2.32-2.04 (m, 1H), 1.99-1.92 (m, 1H,) 1.45 (s, 9H), 1.29 (s, 9H).

### Step 6: Preparation of 2(R)-phenylmethyl-4(S)-(t-butyldimethylsilyloxy)-5-(1-(4-(1,1-dimethylethoxycarbonylamino)))-2(S)-N-(t-butylcarboxamido)-piperazinyl))-pentaneamide

To 25.50g (52.50 mmol) of dihydro-5(S)-(4-(1,1-dimethylethoxycarbonylamino))-2(S)-N-(t-butylcarboxamido)-piperazinyl)methyl)-3(R)-phenylmethyl-3(2H)-furanone dissolved in 120 mL DME cooled to 0°C was added a solution of 60 mL of water and 1.512g (63.01 mmol) of lithium hydroxide. After 0.5 hours the reaction was quenched with the addition of 10% HCl until pH 6 and the solution was concentrated in vacuo. The residue was dissolved in 50 mL water and extracted with EtOAc (4 x 75 mL) and the organic layers were washed with water (1 x 20 mL), brine (1 x 20 mL). The aqueous was back extracted with EtOAc (2 x 75 mL) and the combined organic layers were dried over MgSO₄ and concentrated to provide a yellow solid. This crude product was dissolved in 100 mL of DMF and 17.87g (0.262 mol) of imidazole was added, cooled to 0°C and then 31.50g (0.21 mol) of t-butyldimethylsilyl chloride was added. This stirred 1 hour at 0°C and was then warmed to room temperature. After 20 hours the reaction was quenched with 10 mL methanol and concentrated to half the volume. 100 mL of pH 7 buffered water was added and the aqueous was extracted with EtOAc (4 x 100 mL), the combined organic layers were washed with 10% HCl (2 x 50 mL), water (3 x 75 mL), and brine (1 x 50 mL), dried over MgSO₄ and concentrated to obtain the title compound. This material was used directly in the next step.

### Step 7: Preparation of N-(2(R)-hydroxy-1(S)-indanyl)-2(R)-phenylmethyl-4(S)-(t-butyldimethylsilyloxy)-5-(1-(4-(1,1-dimethylethoxycarbonylamino)))-2(S)-N-(t-butylcarboxamido)-piperazinyl))-pentaneamide

To 27.0g (0.0446mol) of the crude material from step 6 dissolved in 180 mL of DMF and cooled to 0°C was added 8.98g (0.0468 mol) of EDC, 6.32g (0.0468 mol) of HOBt, and 7.31g (0.049 mol) aminohydroxy indane. Triethylamine (6.52 mL, 0.0468 mol) was added and the reaction stirred at 0°C for 2 hours, room temperature for 16 hours and was quenched by diluting with 500 mL of EtOAc. The organic layer was washed with 10% HCl (2 x 100 mL), saturated NaHCO₃ (1 x 100 mL), water (3 x 150 mL), brine (1 x 75 mL), dried over MgSO₄ and concentrated to yield the title compound as a white foam.
¹H NMR (400 MHz, CDCl₃) δ 7.4-7.17 (m, 9H), 6.51 (br s, 1H), 5.79 (br s, 1H), 5.23 (m, 1H), 4.23 (br s, 1H), 4.06 (m, 1H), 3.96-3.84 (m, 2H), 3.07-2.78 (m, 8H), 3.65 (dd, J=9.6 and 4.1 Hz, 1H), 2.56-2.44 (m, 2H), 2.29 (dd, J=12.0 and 4.5 Hz, 1H), 2.17-2.09 (m, 1H), 1.79 (br s, 1H), 1.44 (s, 9H), 1.35 (s, 9H), 1.10 (s, 1H), 0.84 (s, 9H), 0.12 (s, 3H), 0.08 (s, 3H).

### Step 8: Preparation of N-(2(R)-hydroxy-1(S)-indanyl)-2(R)-phenylmethyl-4(S)-(hydroxy)-5-(1-(4-(1,1-dimethylethoxycarbonylamino)))-2(S)-N-(t-butylcarboxamido)-piperazinyl))pentaneamide

To 32.20g (0.0437 mol) of N-(2(R)-hydroxy-1-(S)-indanyl)-2(R)-phenylmethyl-4(S)-(t-butyldimethylsilyloxy)-5-(1-(4-(1,1-dimethylethoxycarbonylamino)))-2(S)-N-(t-butylcarboxamido)-piperazinyl))-pentaneamide was added 437 mL (0.437 mol) of tetrabutylammonium fluoride (1.0M solution in THF, Aldrich). The reaction stirred for 18 hours and was then concentrated to 200 mL and diluted with 700 mL of EtOAc. This was washed with water (2 x 100 mL), brine (1 x 50 mL) and the aqueous layers were back extracted with EtOAc (2 x 200 mL). The combined organic layers were dried over MgSO₄ and concentrated to an oil. Purification via flash column chromatography (120 x 150 mm column, gradient elution CH₂Cl₂: CHCl₃/saturated with NH₃: methanol, increasing methanol from 1%, 1.5%, 2%) afforded the title compound as a white foam.
¹H NMR (400 MHz, CDCl₃) δ 7.31-7.11 (m, 9H), 6.41 (br s, 1H), 6.23 (d, J=8.6 Hz, 1H), 5.25 (dd, J=8.6 and 4.7Hz, 1H), 4.21 (m, 1H), 3.83-3.82 (m, 2H), 3.78-3.61 (m, 2H), 3.22-3.19 (m, 2H), 3.03-2.78 (m, 8H), 2.62-2.58 (m, 1H), 2.41-2.35 (m, 2H), 2.04-2.02 (m, 1H), 1.57-1.50 (m, 1H), 1.45 (s, 9H), 1.32 (s, 9H).

### Step 9: Preparation of N-(2(R)-hydroxy-1(S)-indanyl)-2(R)-phenylmethyl-4(S)-(hydroxy)-5-(1-(2(S)-N-(t-butylcarboxamido)-piperazinyl)-pentaneamide

To 21.15g (0.034 mol) of N-(2(R)-hydroxy-1(S)-indanyl)-2(R)-phenylmethyl-4(S)-(hydroxy)-5-(1-(4-(1,1-dimethylethoxycarbonylamino)))-2(S)-N-(t-butylcarboxamido)-piperazinyl))-pentaneamide dissolved in 350 mL of methylene chloride and cooled to 0°C was added 22.43 mL (0.204 mol) 2,6-lutidine and then 32.85 mL (0.170 mol) of trimethylsilyltriflate over 5 minutes. After 0.5 hours the reaction was quenched with 10% HCl (80 mL) and this stirred 0.5 hours. To this was added 100 mL of saturated NaHCO₃ and then solid NaHCO₃ until pH 8. The aqueous layer was then extracted with EtOAc (4 x 100 mL) and the combined organic layers were washed with water (1 x 50 mL), brine (1 x 75 ml), dried over MgSO₄ and concentrated. The residue was purified via column chromatography (120 x 150 mm column, gradient elution CH₂Cl₂:CHCl₃ saturated with NH₃: MeOH, slowly increasing methanol 2%, 3%, 4%, 5%, 6%, to 10%). This provided the title product as a white foam.
¹H NMR (400 MHz, CDCl₃) δ 7.53 (s, 1H), 7.29-7.09 (m, 9H), 6.52 (d, J=8.3 Hz, 1H), 5.24 (dd, J=8.2 and 4.9 Hz, 1H), 4.23 (dd, J=4.7 and 4.03 Hz, 1H), 4.25-4.00 (br s, 1H), 3.83-3.81 (m, 1H), 3.03-2.88 (m, 4H), 2.82-2.73 (m, 7H), 2.50-1.60 (br s, 2H), 2.45 (d, J=6.2 Hz, 2H), 2.32-2.29 (m, 1H), 1.98 (m, 1H), 1.51(m, 1H), 1.33 (s, 9H).

### Step 10: Preparation of N-(2(R)-hydroxy-1(S)-indanyl)-2(R)-phenylmethyl-4(S)-hydroxy-5-(1-(4-(3-pyridylmethyl)-2(S)-N'-(t-butylcarboxamido)piperazinyl))-pentaneamide

To 10.0g (0.019 mol) of N-(2(R)-hydroxy-1(S)-indanyl)-2(R)-phenylmethyl-4(S)-hydroxy)-5-(1(-2(S)-N-(t-butylcarboxamido)-piperazinyl)-pentaneamide and 3.45g (0.021 mol) of 3-picolyl chloride dissolved in 40 mL of DMF was added 5.85 mL (0.042 mol) of triethylamine. After 3 hours an additional 0.313g of 3-picolyl chloride was added. After an additional 2 hours the reaction was diluted with 400 mL of EtOAc and washed with water (3 x 75 mL), brine (1 x 100 mL), dried over MgSO₄ and concentrated. The residue was triturated with 30 mL of EtOAc and the resulting white precipitate was collected. Further recrystallization from EtOAc provided the title product (mp 167.5-168°C).

### EXAMPLE 16

Employing substantially the same procedure as described in Example 15, but treating the N-(2(R)-hydroxy-1(S)-indanyl)-2(R)-phenylmethyl-4(S)-hydroxy-5-(1-(2(S)-N'-(t-butylcarboxamido)-piperazinyl))-pentaneamide used therein (compound (i) below) with the alkylating agent (ii) indicated below in place of the 3-picolyl chloride used in Step 10 therein, the following products defined by formula (iii) were made:

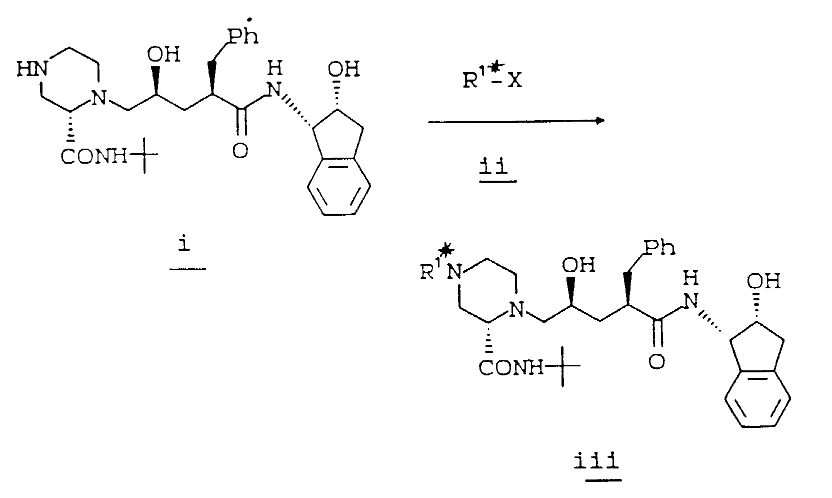

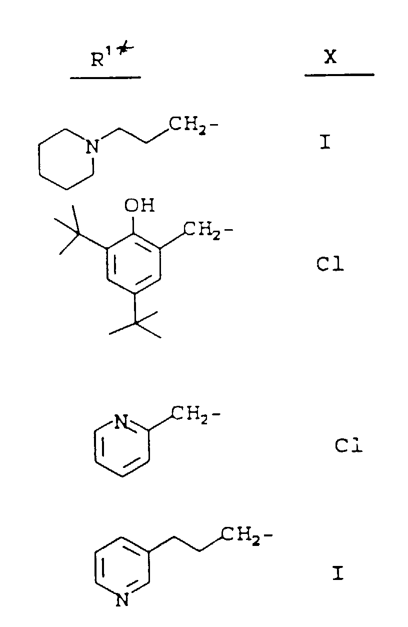

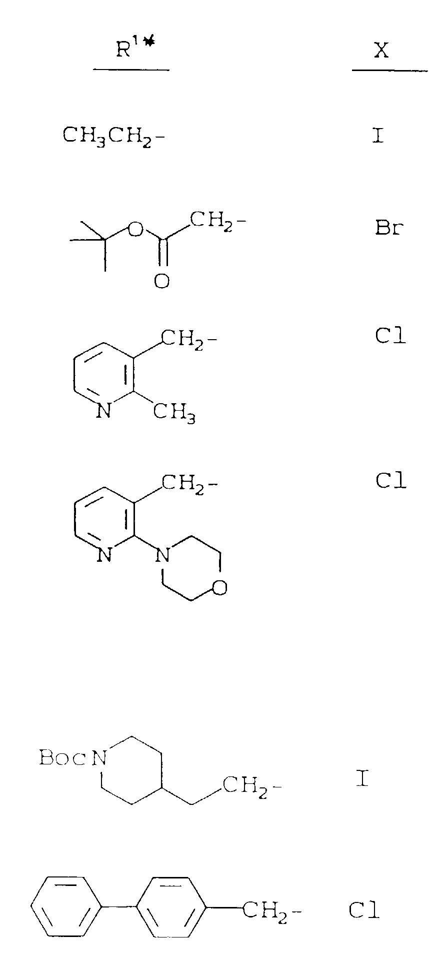

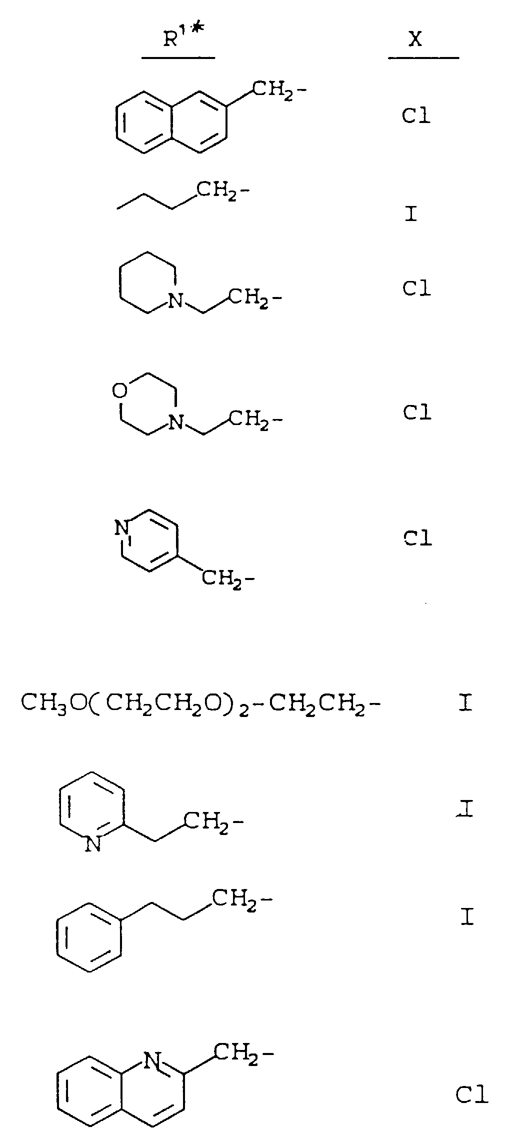

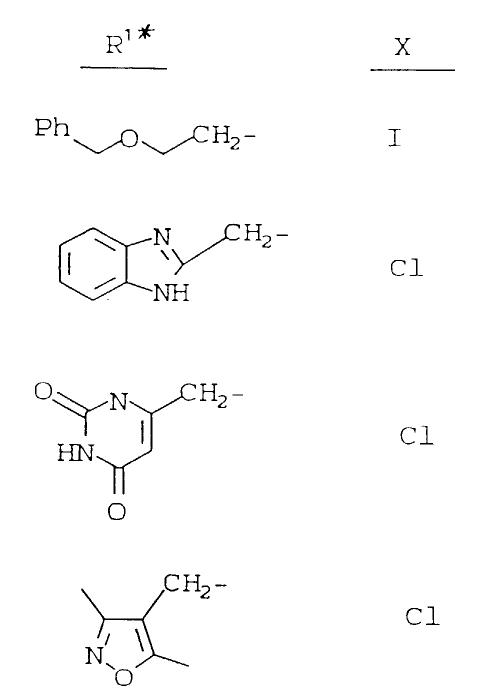

### EXAMPLE 17

### Preparation of dihydro-5(S)-(tert-butyldimethylsilyloxymethyl)-3(2H)-furanone

To a solution of 3.00g (25.8 mmol) of dihydro-5(S)-(hydroxymethyl)-2(3H)-furanone dissolved in 25 mL of dichloromethane was added 3.51g (51.6 mmol) of imidazole and then 4.67g (31.0 mmol) of tert-butyldimethylsilyl chloride. The reaction stirred at room temperature for 8 hours and was quenched with 2 mL of methanol. The mixture was concentrated to an oil and then diluted with 150 mL of ether and washed with 5% HCl (2 x 10 mL), saturated NaHCO₃ (1 x 10 mL), water (1 x 10 mL), and brine (1 x 10 mL), dried over MgSO₄ and concentrated. The residue was purified by flash chromatography (40 x 150 mm column, gradient elution, hexanes:ethyl/acetate 5:1 to 4:1) to afford the product as a clear oil.
¹H NMR (300 MHz, CDCl₃) δ 4.68-4.60 (m, 1H), 3.89 (dd, J=3.3 and 11.3 Hz, 1H), 3.71 (dd, J=3.2 and 5411.3 Hz,1H), 2.71-2.45 (m, 2H), 2.35-2.16 (m, 2H), 0.91 (s, 9H), 0.10 (s, 3H), 0.09 (s, 3H).

## Claims

1. A compound of the formula: 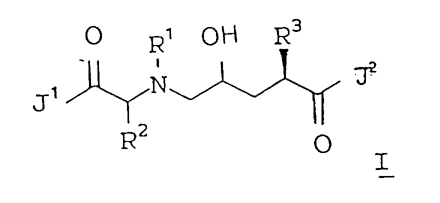 wherein
R is hydrogen or C₁₋₄ alkyl;
R¹ and R² can be joined together to form with the nitrogen to which R¹ is attached a 3 to 10 membered monocyclic or bicyclic saturated ring system which consists of the nitrogen to which R¹ is attached and form 2 to 9 carbon atoms, and is unsubstituted or substituted with
1) -W-aryl, or
2) 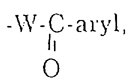 wherein W is -O-, -S- or -NH-; or
R¹ and R² can be joined together to form with the nitrogen to which R¹ is attached a 3 to 10 membered monocyclic or bicyclic saturated ring system which consists of the nitrogen to which R¹ is attached, form 1 to 8 carbon atoms and one of 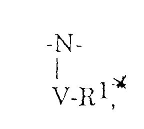
wherein V is absent or 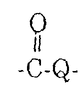 or -SO₂-Q-,
R¹* is
1) hydrogen,
2) -C₁₋₄ alkyl unsubstituted or substituted with one or more of
a) halo,
b) hydroxy,
c) C₁₋₃ alkoxy,
d) aryl unsubstituted or substituted with one or more of C₁₋₄alkyl, hydroxy or aryl,
e) -W-aryl or -W-benzyl, wherein W is -O-, -S-, or -NH-,
f) a 5-7 membered cycloalkyl group unsubstituted or stibstituted with one or more of
i) halo
ii) hydroxy,
iii) C₁₋₃ alkoxy, or
iv) aryl,
g) heterocycle unsubstituted or substituted with one or more of hydroxy, C₁₋₄alkyl optionally substituted with hydroxy, or Boc,
h) 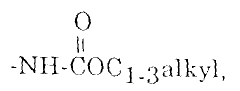
i) 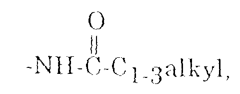
j) -NH-SO₂C₁₋₃alkyl,
k) -NR₂,
l) -COOR, or
m) -((CH₂)ₘO)ₙR wherein m is 2-5 and n is zero, 1, 2 or 3, or
3) aryl, unsubstituted or substituted with one or more of
a) halo,
b) hydroxy,
c) -NO₂ or -NR₂,
d) C₁₋₄alkyl,
e) C₁₋₃ alkoxy, unsubstituted or substituted with one or more of -OH or C₁₋₃ alkoxy,
f) -COOR,
g) 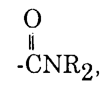
h) -CH₂NR₂,
i) 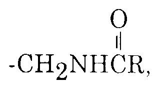
j) -CN,
k) -CF₃,
l) 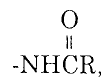
m) aryl C₁₋₃ alkoxy,
n) aryl,
o) -NRSO₂R,
p) -OP(O)(ORₓ)₂, or
q) -R⁵, as defined below;
and wherein Q is absent or -O-, -NR-, or heterocycle optionally substituted with -C₁₋₄alkyl,
R³ is benzyl, unsubstituted or substituted with one or more of (1) hydroxy, (2) C₁₋₃ alkoxy substituted with one or more of -OH or (3) 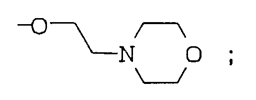
Rₓ is H or aryl;
_{R}5 is
1) -W-(CH₂)ₘ-NR⁶R⁷
wherein W is as defined above,
m is 2-5, and
R⁶ and R⁷ are independently
a) hydrogen,
b) C₁₋₆ alkyl, unsubstituted or substituted with one or more of
i) C₁₋₃ alkoxy,
ii) -OH, or
iii) -NR₂,
c) the same or different and joined together to form a 5-7 member heterocycle, such as morpholino, containing up to two additional heteroatoms selected from 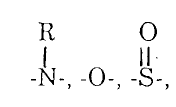 -S-, or -SO₂-, the heterocycle optionally substituted with C₁₋₄ alkyl, or
d) aromatic heterocycle unsubstituted or substituted with one or more of
i) C₁₋₄ alkyl, or
ii) -NR₂
2) -(CH₂)_{q}-NR⁶R⁷ wherein q is 1-5, and R⁶ and R⁷ are defined above, except that R⁶ or R⁷ are not H or unsubstituted C₁₋₆ alkyl, or
3) benzofuryl, indolyl, azacycloalkyl, azabicyclo C₇₋₁₁ cycloalkyl, or benzopiperidinyl, unsubstituted or substituted with C₁₋₄ alkyl;
J¹ is -NH-C₁₋₄alkyl; and
J² is 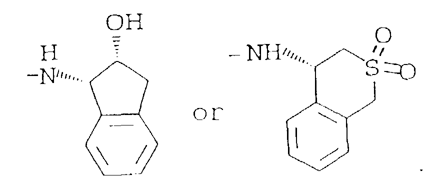 or a pharmaceutically acceptable salt thereof.

2. A compound according to claim 1 of formula 27: 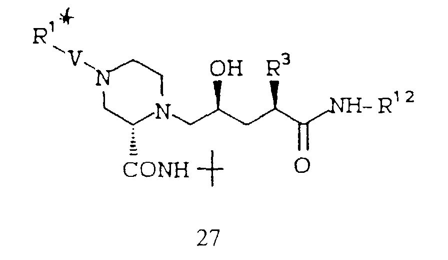 wherein R¹*, R³ and R¹² are as defined in claim 1 except that when V is absent or represents -SO₂-Q- then R¹* is not hydrogen or a group, other than an aryl group, with a free hydroxy substituent, or a pharmaceutically acceptable salt thereof.

3. A compound selected from
N-(2(R)-hydroxy-1(S)-indanyl)-2(R)-phenylmethyl-4(S)-hydroxy-5-(2-(3(S)-N'-(t-butylcarboxamido)-(4aS,8aS)-decahydroisoquinoline)yl)-pentaneamide,
N-(2(R)-hydxoxy-1(S)-indanyl)-2(R)-phonylmethyl-4(S)-hydroxy-5-(1-(4-carbobenzyloxy-2(S)-N'-(t-butylcarboxamido)-piperazinyl))-pentaneamide,
N-(2(R)-hydroxy-1(S)-indanyl)-2(R)-((4-(2-(4-morpholinyl)ethoxy)phenyl)methyl)-4(S)-hydroxy-5-(2-(3(S)-N'-(t-butylcarboxamido)-(4aS,8aS)-decahydroisoquinoline)yl)-pentaneamide,
N-(2(R)-hydroxy-1(S)-indanyl)-2(R)-((4-(2-(4-morpholinyl)ethoxy)phenyl)methyl)-4(S)-hydroxy-5-(1-(4-carbobenzyloxy-2(S)-N'-(t-butylcarboxamido)piperazinyl))-pentaneamide,
N-(2(R)-hydroxy-1(S)-indanyl)-2(R)-((4-((2-hydroxy)-ethoxy)phenyl)methyl)-4(S)-hydroxy-5-(2-(3(S)-N'-(t-butylcarboxamido)-(4aS,8aS)-decahydroisoquinoline)yl)-pentaneamide,
N-(2(R)-hydroxy-1-(S)-indanyl)-2(R)-((4-((2-hydroxy)-ethoxy)phenyl)methyl)-4(S)-hydroxy-5-(1-(4-carbobenzyloxy-2(S)-N'-(t-butylcarboxamido)piperazinyl))-pentaneamide,
N-(4(S)-3,4-dihydro-1H-2,2-dioxobenzothiopyranyl)-2(R)-phenylmethyl-4(S)-hydroxy-5-(2-(3(S)-N'-(t-butylcarboxamido)-(4aS,8aS)-decahydroisoquinoline)yl)pentaneamide,
N-(4(S)-3,4-dihydro-1H-2,2-dioxobenzothiopyranyl)-2(R)-phenylmethyl-4(S)-hydroxy-5-(1-(4-carbobenzyloxy-2(S)-N'-(t-butylcarboxamido)-piperazinyl))pentaneamide,
N-(2(R)-hydroxy-1(S)-indanyl)-2(R)-phenylmethyl-4(S)-hydroxy-5-(1-(4-(3-pyridylmethyl)-2(S)-N'-(t-butylcarboxamido) -piperazinyl))-pentaneamide;
or a pharmaceutically acceptable salt thereof.

4. The compound of the formula: 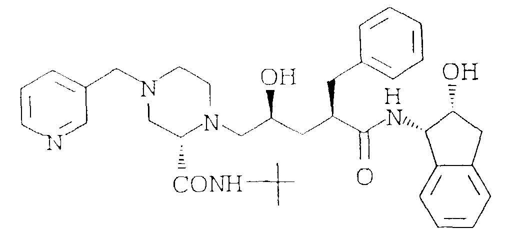 or a pharmaceutically acceptable salt thereof.

5. A pharmaceutical composition comprising a compound as claimed in Claims 1, 2 or 3 and a pharmaceutically acceptable carrier.

6. A composition as claimed in Claim 5 in the form of a unit formulation adapted for oral administration.

7. A composition as claimed in Claim 5 or 5 which comprises a compound as claimed in Claim 3.

8. The use of a compound as claimed in Claims 1 to 4 for the preparation of a medicament useful for the treatment of AIDS in a patient receiving AIDS therapy.

9. The use of a compound of any of Claims 1 to 4 for the preparation of a medicament for preventing or treating infection by HIV in a patient.

10. The use of a compound of any of Claims 1 to 4 for the preparation of a medicament useful for inhibiting HIV protease in a patient receiving HIV protease therapy.

11. A process for the preparation of a compound of formula 27: 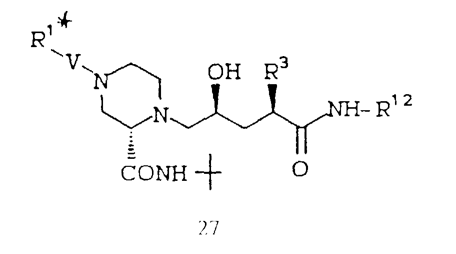 wherein R¹*, R³ and R¹² are as defined in Claim 1 except that when V is absent or represents -SO₂-Q- then R¹* is not hydrogen or a group, other than an aryl group, with a free hydroxy substituent, which comprises reacting a compound of formula 28: 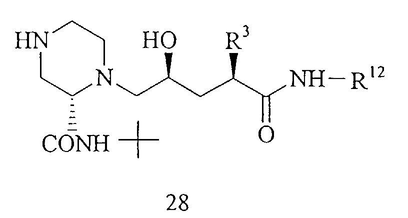 wherein R³ and R¹² are as defined above with a compound of formula R¹*-X or R¹*SO₂Cl, where R¹* is as defined above and X is Cl, Br or I, or using standard amide coupling techniques.

12. A process according to claim 11 where the compound of formula 28 is reacted with a compound of formula R¹*-X.

13. A process according to claim 11 or 12 where R³ is (R)-benzyl and R¹² is 2(R)-hydroxy-1(S)-indanyl.

14. A process according to claim 11, 12 or 13 where the compound of formula 28 is N-2(2(R)-hydroxy-1(S)-indanyl)-2(R)-phenylmethyl-4(S)-hydroxy-5-(1-(2(S)-N-(t-butylcarboxamido)-piperazinyl))-pentaneamide.

15. A process for the preparation of a compound of Claim 4 which which comprises the reaction of picolyl chloride, bromide or iodide with N-2(2(R)-hydroxy-1(S)-indanyl)-2(R)-phenylmethyl-4(S)-hydroxy-5-(1-(2(S)-N-(t-butylcarboxamido)-piperazinyl))-pentaneamide in a solvent.

16. A process as claimed in Claim 15 where the solvent is DMF.

17. N-(2-(R)-hydroxy-1(S)-indanyl)-2(R)-phenylmethyl-4(S)-hydroxy-5-(1-(2(S)-N-(t-butylcarboxamido)piperazinyl))-pentaneamide.

## Patentansprüche

1. Eine Verbindung der Formel: 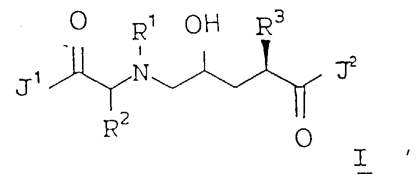 worin
R Wasserstoff oder C₁_₄-Alkyl ist;
R¹ und R² miteinander verbunden sein können, um mit dem Stickstoff, an den R¹ gebunden ist, ein 3- bis 10 gliedriges monocyclisches oder bicyclisches gesättigtes Ringsystem zu bilden, das aus dem Stickstoff, an den R¹ gebunden ist, und 2 bis 9 Kohlenstoffatomen besteht, und das unsubstituiert oder mit
1) -W-Aryl oder
2) 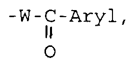 substituiert ist, wobei W -O-, -S- oder -NH- ist; oder
R¹ und R² miteinander verbunden sein können, um mit dem Stickstoff, an den R¹ gebunden ist, ein 3- bis 10 gliedriges monocyclisches oder bicyclisches gesättigtes Ringsystem zu bilden, das aus dem Stickstoff, an den R¹ gebunden ist, 1 bis 8 Kohlenstoffatomen und einem der Reste 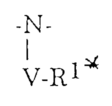 besteht,
worin V nicht vorhanden ist oder 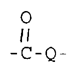 oder -SO₂-Q- ist,
R¹* ist
1) Wasserstoff,
2) -C₁₋₄-Alkyl, unsubstituiert oder substituiert mit einer oder mehreren der Gruppen
a) Halogen,
b) Hydroxy,
c) C₁₋₃-Alkoxy,
d) Aryl, unsubstituiert oder mit einem oder mehreren C₁₋₄-Alkyl, Hydroxy oder Aryl substituiert,
e) -W-Aryl oder -W-Benzyl, worin W -O-, -S- oder -NH- ist,
f) eine 5-7gliedrige Cycloalkylgruppe, unsubstituiert oder substituiert mit einer oder mehreren der Gruppen
i) Halogen,
ii) Hydroxy,
iii) C₁₋₃-Alkoxy oder
iv) Aryl,
g) Heterocyclus, unsubstituiert oder substituiert mit einem oder mehreren Hydroxy, C₁₋₄-Alkyl, gegebenenfalls substituiert mit Hydroxy, oder Boc,
h) 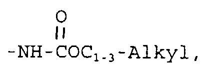
i) 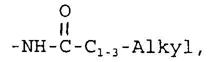
j) -NH-SO₂C₁₋₃-Alkyl,
k) -NR₂,
l) -COOR oder
m) -((CH2)ₘO)ₙR, worin m 2-5 und n null, 1, 2 oder 3 ist, oder
Aryl, unsubstituiert oder substituiert mit einer oder mehreren der Gruppen
a) Halogen,
b) Hydroxy,
c) -NO₂ oder -NR₂,
d) C₁₋₄-Alkyl,
e) C₁-₃-Alkoxy, unsubstituiert oder substituiert mit einem oder mehreren -OH oder C₁_₃-Alkoxy,
f) -COOR,
g) 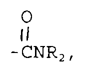
h ) -CH₂NR₂,
i) 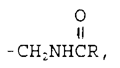
j) -CN,
k) -CF₃,
l) 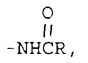
m) Aryl-C₁₋₃-alkoxy,
n) Aryl,
o) -NRSO₂R,
p) -OP(O) (OR_{X})2 oder
q) -R⁵, wie oben definiert;
und worin Q nicht vorhanden ist oder -O-, -NR- oder ein Heterocyclus ist, der gegebenenfalls mit -C₁₋₄-Alkyl substituiert ist;
R³ Benzyl ist, unsubstituiert oder substituiert mit einem oder mehreren der Reste (1) Hydroxy, (2) C₁₋₃-Alkoxy, substituiert mit einem oder mehreren -OH, oder (3) 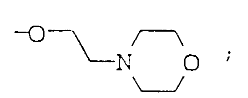
Rₓ H oder Aryl ist;
R⁵ ist
1) -W-(CH₂)ₘ-NR⁶R⁷,
worin W wie oben definiert ist,
m 2-5 ist, und
R⁶ und R⁷ unabhängig voneinander sind
a) Wasserstoff,
b) C₁₋₆-Alkyl, unsubstituiert oder substituiert mit einer oder mehreren der Gruppen
i) C₁₋₃-Alkoxy,
ii) -OH oder
iii) -NR₂,
c) gleich oder verschieden und miteinander verbunden, um einen 5-7gliedrigen Heterocyclus zu bilden, wie z.B. Morpholino, der bis zu zwei zusätzliche Heteroatome enthält, ausgewählt aus 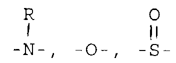 oder -SO₂-, wobei der Heterocyclus gegebenenfalls mit C₁_₄-Alkyl substituiert ist, oder
d) aromatischer Heterocyclus, unsubstituiert oder substituiert mit einer oder mehreren der Gruppen
i) C₁₋₄-Alkyl oder
ii) -NR₂,
2) -(CH₂)_{q}-NR⁶R⁷, worin q 1-5 ist, und R⁶ und R⁷ wie oben definiert sind, außer daß R⁶ oder R⁷ nicht H oder unsubstituiertes C₁₋₆-Alkyl sind, oder
3) Benzofuryl, Indolyl, Azacycloalkyl, Azabicyclo-C₇_₁₁-cycloalkyl oder Benzopiperidinyl, unsubstituiert oder substituiert mit C₁₋₄-Alkyl;
J¹ -NH-C₁₋₄-Alkyl ist; und
J² 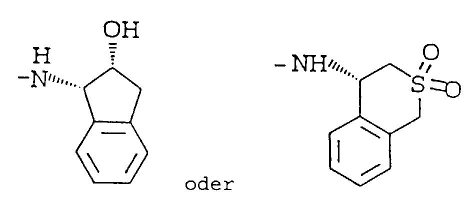 ist, oder ein pharmazeutisch annehmbares Salz davon.

2. Eine Verbindung gemäß Anspruch 1 der Formel 27: 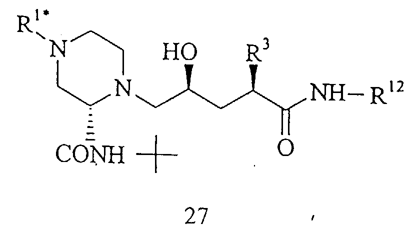 worin R¹*, R³ und R¹² wie in Anspruch 1 definiert sind, außer daß, wenn V nicht vorhanden ist oder -SO₂-Q- bedeutet, R¹ dann nicht Wasserstoff oder eine Gruppe, die keine Arylgruppe ist, mit einem freien Hydroxysubstituenten ist, oder ein pharmazeutisch annehmbares Salz davon.

3. Eine Verbindung, ausgewählt aus
N-(2(R)-Hydroxy-1(S)-indanyl)-2(R)-phenylmethyl-4(s)-hydroxy-5-(2-(3(S)-N'-(t-butylcarboxamido)(4aS,8aS)-decahydroisochinolin)yl)pentanamid,
N-(2(R) -Hydroxy-1(S)-indanyl)-2(R)-phenylmethyl-4(S)-hydroxy-5-(1-(4-carbobenzyloxy-2(S)-N'-(t-butylcarboxamido)-piperazinyl))pentanamid,
N-(2(R) -Hydroxy-1(S)-indanyl)-2(R)-((4-(2-(4-morpholinyl)ethoxy)phenyl)methyl)-4(S)-hydroxy-5-(2-(3(S)-N'-(t-butylcarboxamido)-(4aS,8aS)decahydroisochinolin)yl)pentanamid,
N-(2(R)-Hydroxy-1(S)-indanyl)-2(R)-((4-(2-(4-morpholinyl)ethoxy)phenyl)methyl)-4(S)-hydroxy-5-(1-(4-carbobenzyloxy-2(S)-N'-(t-butylcarboxamido)piperazinyl))pentanamid,
N-(2(R)-Hydroxy-1(S)-indanyl)-2(R)-((4-((2-hydroxy)ethoxy)phenyl)methyl)-4(S)-hydroxy-5-(2-(3(S)-N'-(t-butylcarboxamido)-(4aS,8aS)-decahydroisochinolin)yl)pentanamid,
N-(2(R)-Hydroxy-1(S)-indanyl)-2(R)-((4-((2-hydroxy)ethoxy)phenyl)methyl)-4(S)-hydroxy-5-(1-(4-carbobenzyloxy-2(S)-N'-(t-butylcarboxamido)piperazinyl))pentanamid,
N-(4(S)-3,4-Dibydro-1H-2,2-dioxobenzothiopyranyl)-2(R)-phenylmethyl-4(S)-hydroxy-5-(2-(3(S)-N'-(t-butylcarboxamido)-(4aS,8aS)-decahydroisochinolin)yl)pentanamid,
N-(4(S)-3,4-Dihydro-1H-2,2-dioxobenzothiopyranyl)-2(R)-phenylmethyl-4(S)-hydroxy-5-(1-(4-carbobenzyloxy-2(S)-N'-(t-butylcarboxamido)piperazinyl))pentanamid,
N-(2(R)-Hydroxy-1(S)-indanyl)-2(R)-phenylmethyl-4(S)hydroxy-5-(1-(4-(3-pyridylmethyl)-2(S)-N'-(t-butylcarboxamido)piperazinyl))pentanamid,
oder ein pharmazeutisch annehmbares Salz davon.

4. Die Verbindung der Formel: 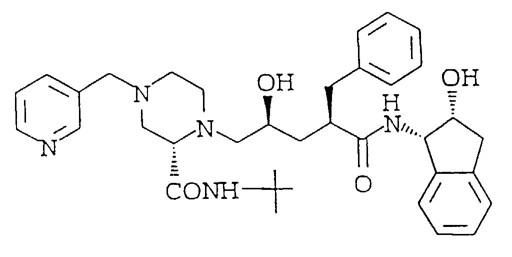 oder ein pharmazeutisch annehmbares Salz davon.

5. Eine pharmazeutische Zusammensetzung, die eine wie in den Ansprüchen 1, 2 oder 3 beanspruchte Verbindung und einen pharmazeutisch annehmbaren Träger enthält.

6. Eine wie in Anspruch 5 beanspruchte Zusammensetzung in der Form einer Einheitsformulierung, die zur oralen Verabreichung hergerichtet ist.

7. Eine wie in Anspruch 5 oder 6 beanspruchte Zusammensetzung, die eine wie in Anspruch 4 beanspruchte Verbindung enthält.

8. Die Verwendung einer wie in den Ansprüchen 1 bis 4 beanspruchten Verbindung zur Herstellung eines Medikaments, das zur Behandlung von AIDS bei einem Patienten, der sich einer AIDS-Therapie unterzieht, geeignet ist.

9. Die Verwendung einer Verbindung nach irgendeinem der Ansprüche 1 bis 4 zur Herstellung eines Medikaments zur Verhinderung oder Behandlung einer HIV-Infektion bei einem Patienten.

10. Die Verwendung einer Verbindung nach irgendeinem der Ansprüche 1 bis 4 zur Herstellung eines Medikaments, das zur Inhibierung von HIV-Protease bei einem Patienten, der sich einer HIV-Proteasetherapie unterzieht, geeignet ist.

11. Ein Verfahren zur Herstellung einer Verbindung der Formel 27: 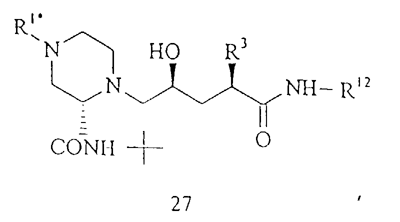 worin R¹*, R³ und R¹² wie hierin zuvor definiert sind, außer daß, wenn V nicht vorhanden ist oder -SO₂-Q- bedeutet, R¹ dann nicht Wasserstoff oder eine Gruppe, die keine Arylgruppe ist, mit einem freien Hydroxysubstituenten ist, umfassend die Umsetzung einer Verbindung der Formel 28: 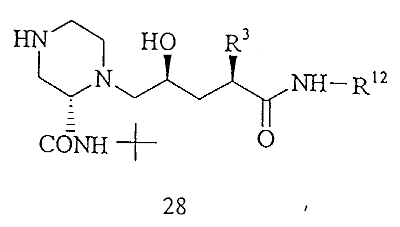 worin R³ und R¹² wie oben definiert sind, mit einer Verbindung der Formel R¹*-x oder R¹*SO₂Cl, worin R¹* wie oben definiert ist und X Cl, Br oder I ist, oder das Anwenden von Standard-Amidkupplungsverfahren.

12. Ein Verfahren gemäß Anspruch 11, bei dem die Verbindung der Formel 28 mit einer Verbindung der Formel R¹*-X umgesetzt wird.

13. Ein Verfahren gemäß Anspruch 11 oder 12, bei dem R³ (R)-Benzyl und R¹² 2(R)-Hydroxy-1(S)-indanyl ist.

14. Ein Verfahren gemäß Anspruch 11, 12 oder 13, worin die Verbindung der Formel 28 N-2(2(R)-Hydroxy-1(S)-indanyl)-2(R)-phenylmethyl-4(S)hydroxy-5-(1-(2(S)-N-(t-butylcarboxamido)piperazinyl))pentanamid ist.

15. Ein Verfahren zur Herstellung einer Verbindung nach Anspruch 4, welches die Reaktion von Picolylchlorid, -bromid oder -iodid mit N-2(2(R)-Hydroxy-1(S)-indanyl)-2(R)-phenylmethyl-4(S)-hydroxy-5-(1-(2(S)-N-(t-butylcarboxamido)piperazinyl))pentanamid in einem Lösungsmittel umfaßt.

16. Ein wie in Anspruch 15 beanspruchtes Verfahren, bei dem das Lösungsmittel DMF ist.

17. N-(2-(R)-Hydroxy-1(S)-indanyl)-2(R)-phenylmethyl-4(S)-hydroxy-5-(1-(2(S)-N-(t-butylcarboxamido)piperazinyl))pentanamid.

## Revendications

1. Composé de formule: 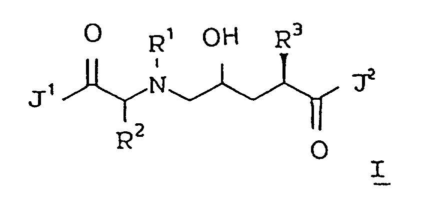 dans laquelle
R est un atome d'hydrogène ou un groupe alkyle en C₁ à C₄;
R¹ et R² peuvent être réunis ensemble pour former, avec l'atome d'azote auquel R¹ est fixé, un système monocyclique ou bicyclique saturé de 3 à 10 éléments qui se compose de l'atome d'azote auquel R¹ est fixé et de 2 à 9 atomes de carbone, et est non substitué ou substitué par
1) -W-aryle, ou
2) 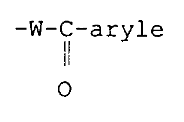 où W représente -O-, -S- ou -NH-; ou bien
R¹ et R² peuvent être réunis ensemble pour former, avec l'atome d'azote auquel R¹ est fixé, un système monocyclique ou bicyclique saturé de 3 à 10 éléments qui se compose de l'atome d'azote auquel R¹ est fixé, de 1 à 8 atomes de carbone et d'un parmi 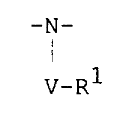
où V est absent ou représente 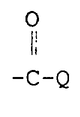 ou SO₂-Q-
R¹ représente
1) un atome d'hydrogène
2) un groupe alkyle en C₁ à C₄ non substitué ou substitué par un ou plusieurs parmi
a) un atome d'halogène
b) un groupe hydroxy,
c) un groupe alcoxy en C₁ à C₃
d) un groupe aryle non substitué ou substitué par un ou plusieurs parmi les groupes alkyle en C₁ à C₄, hydroxy ou aryle,
e) -W-aryle ou -W-benzyle dans lequel W représente -O-, -S-, ou -NH-,
f) Un groupe cycloalkyle à 5 ou 7 éléments non substitué ou substitué par un ou plusieurs parmi
i) un atome d'halogène,
ii) un groupe hydroxy,
iii) un groupe alcoxy en C₁ à C₃, ou
iv) un groupe aryle,
g) un hétérocycle non substitué ou substitué par un ou plusieurs parmi un groupe hydroxy, un groupe alkyle en C₁ à C₄ éventuellement substitué par un groupe hydroxy, ou Boc,
h) 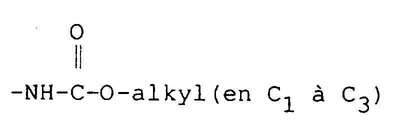
i) 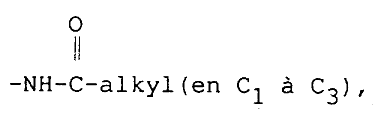
j) -NH-SO₂-alkyl(en C₁ à C₃),
k) -NR₂
l) -COOR, ou
m) -((CH₂)ₘO)ₙR dans lequel m vaut de 2 à 5 et n vaut 0, 1, 2 ou 3, ou
3) un groupe aryle non substitué ou substitué par un ou plusieurs parmi
a) un atome d'halogène,
b) un groupe hydroxy,
c) -NO₂ ou -NR₂,
d) un groupe alkyle en C₁ à C₄
e) un groupe alcoxy en C₁ à C₃, non substitué ou substitué par un ou plusieurs parmi -OH ou un groupe alcoxy en C₁ à C₃
f) -COOR,
g) 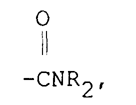
h) -CH₂NR₂,
i) 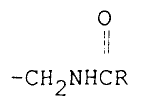
j) -CN,
s
k) -CF₃
l) 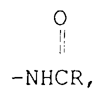
m) arylalcoxy(en C₁ à C₃)
n) aryle,
o) -NRSO₂R,
p) OP(O)(ORₓ)₂, ou
q) -R⁵, tel que défini ci-dessous; et où Q est absent ou représente -O-, -NR-, ou un hétérocycle éventuellement substitué par un groupe alkyle en C₁ à C₄,
R³ est un groupe benzyle, non substitué ou substitué par un ou plusieurs parmi
(1) un groupe hydroxy,
(2) un groupe alcoxy en C₁ à C₃ substitué par un ou plusieurs de -OH ou (3) 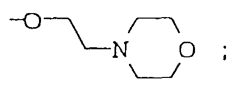
Rₓ est un atome d'hydrogène ou un groupe aryle
R⁵ représente
1) -W(CH₂)ₘ-NR⁶R⁷
où W est tel que défini ci-dessus
m vaut de 2 à 5, et
R⁶ et R⁷ représentent indépendamment l'un de l'autre
a) un atome d'hydrogène
b) un groupe alkyle en C₁ à C₆, non substitué ou substitué avec un ou plusieurs parmi
i) un groupe alcoxy en C₁ à C₃
ii) -OH, ou
iii) -NR₂,
c) identiques ou différents et réunis ensemble pour former un hétérocycle à 5 à 7 éléments, tels que morpholino, contenant jusqu'à deux hétéroatomes supplémentaires choisis parmi 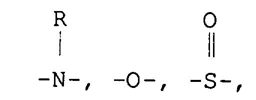 ou -SO₂-, l'hétérocycle éventuellement substitué par un groupe alkyle en C₁ à C₄, ou
d) un hétérocycle aromatique non substitué ou substitué par un ou plusieurs parmi
i) un groupe alkyle en C₁ à C₄, ou
ii) -NR₂
2) -(CH₂)_{q}-NR⁶R⁷ où q vaut de 1 à 5, et R⁶ et R⁷ sont tels que définis ci-dessus, sauf que R⁶ ou R⁷ ne représentent pas un atome d'hydrogène ou un groupe alkyle en C₁ à C₆ non substitué, ou
3) un groupe benzofuryle, indolyle, azacycloalkyle, azabicyclocycloalkyl(en C₇ à C₁₁), ou benzopipéridinyle, non substitué ou substitué par un groupe alkyle en C₁ à C₄;
J¹ représente un groupe -NH-alkyl(en C₁ à C₄); et
J² représente 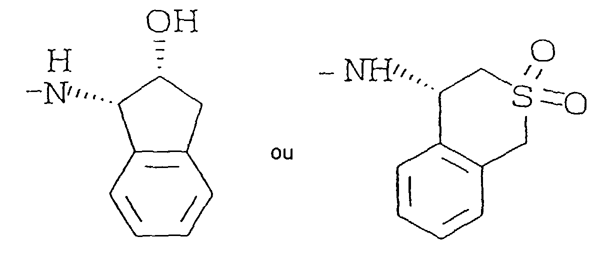 ou un sel de celui-ci acceptable sur le plan pharmaceutique.

2. Composé selon la revendication 1 de formule 27: 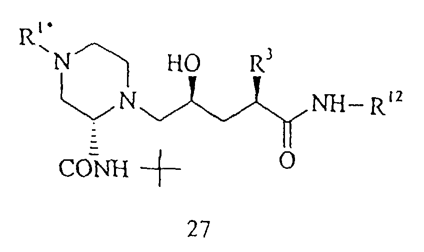 dans laquelle R¹*, R³ et R¹² sont tels que définis selon la revendication 1 sauf que lorsque V est absent ou représente -SO₂Q- alors R¹ ne représente pas un atome d'hydrogène ou un groupe, autre qu'un groupe aryle, avec un substituant hydroxy libre, ou un sel de celui-ci acceptable sur le plan pharmaceutique.

3. Composé choisi parmi:
- le N-(2(R)-hydroxy-1(S)-indanyl)-2(R)-phénylméthyl-4(S)-hydroxy-5-(2-(3(S)-N'-(t-butylcarboxamido)-(4aS,8aS)-décahydroisoquinoline)yl)-pentaneamide,
- le N-(2(R)-hydroxy-1(S)-indanyl)-2(R)-phénylméthyl-4(S)-hydroxy-5-(1-(4-carbobenzyloxy-2(S)-N'-(t-butylcarboxamido)-pipérazinyl))-pentaneamide,
- le N-(2-(R)-hydroxy-1(S)-indanyl)-2(R)-((4-(2-(4-morpholinyl)éthoxy)phényl)méthyl)-4(S)-hydroxy-5-(2-(3(S)-N'-(t-butylcarboxamido)-(4aS,8aS)-décahydroisoquinoline)yl)-pentaneamide,
- le N-(2(R)-hydroxy-1(S)-indanyl)-2(R)-((4-(2-4-morpholinyl)éthoxy)phényl)méthyl)-4(S)-hydroxy-5-1-(4-carbobenzyloxy-2-(S)-N'-(t-butylcarboxamido)-pipérazinyl))pentaneamide,
- le N-(2(R)-hydroxy-1(S)-indanyl)-2-(R)-((4-((2-hydroxy)éthoxy)phényl)méthyl)-4-(S)-hydroxy-5-(2-(3(S)-N'-(t-butylcarboxamido)-(4aS,8aS)-décahydroisoquinoline)-yl)pentaneamide,
- le N-(2(R)-hydroxy-1(S)-indanyl)-2(R)-((4-((2-hydroxy)éthoxy)phényl)méthyl)-4(S)-hydroxy-5-(1-(4-carbobenzyloxy-2(S)-N'-(t-butylcarboxamido)-pipérazinyl))-pentaneamide,
- le N-(4(S)-3,4-dihydro-1H-2,2-dioxobenzothiopyranyl)-2(R)-phénylméthyl-4(S)-hydroxy-5-(2-(3(Si-N'-(t-butylcarboxamido)-(4aS,8aS)-décahydroisoquinoline)yl-pentaneamide,
- le N-(4(S)-3,4-dihydro-1H-2,2-dioxobenzothiopyranyl)2(R)-phénylméthyl-4(S)-hydroxy-5-(1-(4-carbobenzyl-oxy-2(S)-N'-(t-butylcarboxamido)-pipérazinyl))-pentaneamide,
- le N-(2(R)-hydroxy-1(S)-indanyl)-2(R)-phénylméthyl-4(S)-hydroxy-5-(1-(4-(3-pyridylméthyl)-2(S)-N'-(t-butylcarboxamido)-pipérazinyl))-pentaneamide;
ou un de leurs sels acceptable sur le plan pharmaceutique.

4. Composé de formule: 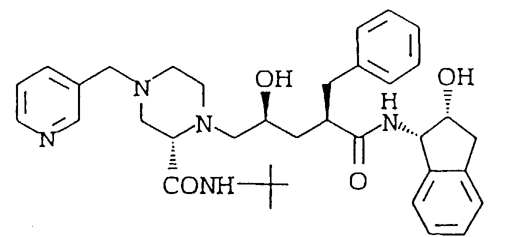 ou un de ses sels acceptable sur le plan pharmaceutique.

5. Composition pharmaceutique comprenant un composé selon les revendications 1, 2 ou 3 et un véhicule acceptable sur le plan pharmaceutique.

6. Composition selon la revendication 5, sous forme d'une formulation d'unité adaptée à une administration par voie orale.

7. Composition selon la revendication 5, ou 5 qui comprend un composé selon la revendication 3.

8. Utilisation d'un composé selon les revendications 1 à 4 pour la préparation d'un médicament utile pour le traitement du SIDA chez un patient recevant une thérapie pour le SIDA.

9. Utilisation d'un composé selon l'une quelconque des revendications 1 à 4 pour la préparation d'un médicament pour la prévention ou le traitement d'infection par HIV chez un patient.

10. Utilisation d'un composé selon l'une quelconque des revendications 1 à 4 pour la préparation d'un médicament utile pour inhiber la HIV-protéase chez un patient recevant une thérapie contre la HIV-protéase.

11. Procédé pour la préparation d'un composé de formule 27: 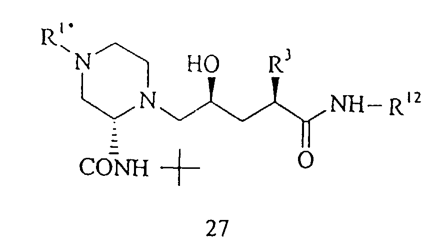 où R¹*, R³ et R¹² sont tels que définis ci-dessus sauf que, lorsque V est absent ou représente -SO₂-Q- alors R¹ n'est pas un atome d'hydrogène ou un groupe, autre qu'un groupe aryle, avec un substituant hydroxy libre, qui consiste à faire réagir un composé de formule 28: 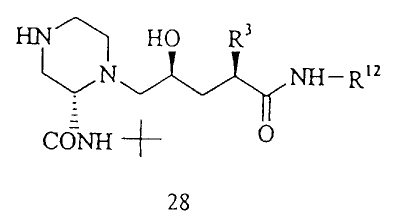 où R³ et R¹² sont tels que définis ci-dessus avec un composé de formule R¹* -X ou R¹* SO₂Cl, lorsque R¹* est tel que défini ci-dessus et X représente Cl, Br ou I, ou en utilisant des techniques de couplage d'amide classiques.

12. Procédé selon la revendication 11 dans lequel le composé de formule 28 est réagi avec un composé de formule R¹* -X

13. Procédé selon la revendication 11 ou 12 dans lequel R³ est un groupe (R)-benzyle et R¹² est un groupe 2(R)-hydroxy-1(S)-indanyle.

14. Procédé selon la revendication 11, 12 ou 13 dans lequel le composé de formule 28 est le N-2(2(R)-hydroxy-1(S)-indanyl)-2(R)-phénylméthyl-4(S)-hydroxy-5-(1-(2(S)-N-(t-butylcarboxamido)-pipérazinyl-pentaneamide.

15. Procédé pour la préparation d'un composé selon la revendication 4 qui consiste à faire réagir le chlorure, bromure ou iodure de picolyle avec le N-2(2(R)-hydroxy-1(S)-indanyl)-2(R)-phénylméthyl-4(S)-hydroxy-5-(1-(2(S)-N-(t-butylcarboxamido)-pipérazinyl))-pentaneamide dans un solvant.

16. Procédé selon la revendication 15 dans lequel le solvant est DMF.

17. Le N-(2-(R)-hydroxy-1(S)-indanyl)-2(R)-phénylméthyl-4(S)-hydroxy-5-(1-(2(S)-N-(t-butylcarboxamido)pipérazinyl))-pentaneamide.
